# EUROPEAN PATENT APPLICATION

(11) **EP 1 655 022 A1**
(43) Date of publication of application: **10.05.2006**
(21) Application number: 04771330.0
(22) Date of filing: 30.07.2004
(51) Int. Cl.: A61K 9/127, A61K 47/24, A61K 47/26, A61K 47/28, A61K 47/36, A61K 47/42, A61K 49/00, A61P 29/00

(54) **REMEDY OR DIAGNOSTIC FOR INFLAMMATORY DISEASE CONTAINING TARGET-DIRECTING LIPOSOME**

(30) Priority: 01.08.2003 JP 2003285422; 29.10.2003 JP 2003369494
(71) Applicant: National Institute of Advanced Industrial Science and Technology, Tokyo 100-8921 (JP)
(72) Inventor: YAMAZAKI, Noboru, Tsukuba-shi, Ibaraki, 3058565 (JP); SURUSHIMA, Hideo, Tsukuba-shi, Ibaraki, 3058565 (JP); OOGURO, Nobuyuki, Tsukuba-shi, Ibaraki, 3058565 (JP)
(74) Representative: HOFFMANN EITLE
(86) International application number: PCT/JP2004/011329
(87) International publication number: WO 2005/011633

(57) **Abstract**

There is provided targeting drug delivery system (DDS) nanoparticles that can be accumulated in target tissues such as inflammatory sites of inflammatory diseases and can thus be utilized in a therapeutic or diagnostic DDS for locally delivering drugs or genes to the affected parts. The present invention provides a pharmaceutical composition for the medical treatment or diagnosis of an inflammatory disease comprising a sugar-modified liposome having a sugar chain bound to the membrane of the liposome.

## Description

### Technical Field

The present invention relates to a drug delivery system intended for inflammatory diseases.

### Background Art

The realization of a "drug delivery system (DDS) for delivering drugs or genes intentionally and intensively to cancer cells or target tissues" has been set as one of the specific goals of the U.S. National Nanotechnology Initiative (NNI). The Nanotechnology/Materials Strategy of the Council for Science and Technology Policy in Japan also focuses research on "Medical micro systems/materials, Nanobiology for utilizing and controlling biological mechanisms," and one of the five year R & D targets is "Establishment of basic seeds in health/life-lengthening technologies such as biodynamic materials and pinpoint treatments." However, even in view of these goals the incidence and morbidity of cancers become higher year after year, along with a progressive aging of the population, and a serious need for the development of a targeting DDS material which is a novel treatment material exists. Targeting DDS nano-structured materials for other diseases also come under the spotlight because they have no side effects, and their market size of over 10 trillion yen is anticipated in the near future. Further, it is expected that these materials will be utilized in medical diagnosis as well as medical treatments.

The therapeutic effect of a drug will be achieved only if the drug reaches a specific target region and acts thereupon. If the drug reaches a non-target region, undesirable side effects may result. Thus, the development of a drug delivery system that allows drugs to be used effectively and safely is also desired. In a drug delivery system, the targeting DDS can be defined as a concept of delivering a drug to a "necessary region in a body," in a "necessary amount" and for a "necessary time-period." A liposome is a noteworthy particulate carrier regarded as a representative material for a targeting DDS. While a passive targeting method based on modification of lipid type, composition ratio, size, or surface charge of liposomes has been developed to impart a targeting function to this particle, this method is still insufficient and required to be improved in many respects.

An active targeting method has also been researched in an attempt to achieve a sophisticated targeting function. While the active targeting method referred to as a "missile drug" is conceptually ideal, it has not been accomplished in Japan and abroad, and future developments are expected. This method is designed to provide ligands bonded to the membrane surface of a liposome that will be specifically recognized and bound by a receptor residing on the cell-membrane surface of a target tissue, thereby achieving active targeting. The cell-membrane surface receptor ligands include antigens, antibodies, peptides, glycolipids, and glycoproteins. It is revealing that the sugar chain of glycolipids and glycoproteins bears an important role as an information molecules in various communications between cells, such as in the creation or morphogenesis of tissues, in the proliferation or differentiation of cells, in the biophylaxis or fecundation mechanism, and in the creation and metastasis of cancers.

Further, research on various types of lectins (sugar-recognizing protein) such as selectin, DC-SIGN, DC-SGNR, a C-type lectin including colectin and mannose binding lectin, an I-type lectin including siglec, a P-type lectin including a mannose-6-phosphate receptor, an R-type lectin, an L-type lectin, an M-type lectin, and galectin, which serve as receptors on cell-membrane surfaces of target tissues, has been proposed to serve as receptors for sugar chains having different molecular structures that may be used as noteworthy new DDS ligands (Yamazaki, N., Kojima, S., Bovin, N. V., Andre, S., Gabius, S. and H. -J. Gabius. *Adv. Drug Delivery Rev.* 43:225-244 (2000); Yamazaki, N., Jigami, Y., Gabius, H. -J., and S. Kojima. *Trends in Glycoscience and Glycotechnology* 13:319-329 (2001); http://www.gak.co.jp/TTGG/71PDF/yamazaki.pdf).

Liposomes having ligands bonded to their external membrane surface have been actively researched in order to provide a DDS material for delivering drugs or genes selectively to a target region, such as cancer. While these liposomes bind to target cells in vitro, most of them do not exhibit adequate targeting to intended target cells or tissues in vivo (Forssen, E. and M. Willis. *Adv. Drug Delivery Rev.* 29:249-271 (1998); Takahashi, T. and M. Hashida. *Today's DDS*/*Drug Delivery System,* Iyaku Journal Co., Ltd. (Osaka, Japan), 159-167 (1999)). While some research has been conducted on liposomes incorporating glycolipids having sugar chains, for use as a DDS material, these liposomes were evaluated only in vitro, and little progress has been reported for similar research on liposomes incorporating glycoproteins having sugar chains (DeFrees, S. A., Phillips, L., Guo, L. and S. Zalipsky. *J. Am. Chem. Soc.* 118:6101-6104 (1996); Spevak, W., Foxall, C., Charych, D. H., Dasqupta, F. and J. O. Nagy. *J. Med. Chem.* 39:1918-1020 (1996); Stahn, R., Schafer, H., Kernchen, F. and J. Schreiber. *Glycobiology* 8:311-319 (1998); Yamazaki, N., Jigami, Y., Gabius, H. -J., and S. Kojima. *Trends in Glycoscience and Glycotechnology* 13:319-329 (2001); http://www.gak.co.jp/TTGG/71PDF/yamazaki.pdf). As above, systematic research into liposomes having a wide variety of sugar chains, on the glycolipids or glycoproteins bonded to the liposomes, including preparative methods and in vivo analyses thereof, is pending and represents an important challenge to be progressed in future.

Further, in research on new types of DDS materials, it is an important challenge to develop a DDS material capable of being orally administered in the easiest and cheapest way For example, when a peptide medicine is orally administered, it is subject to enzymolysis and may be only partially absorbed in the intestine due to its water solubility, high molecular weight, and low permeability in the mucosa of small intestine. As an alternative, a ligand-bonded liposome is getting attention as a potential DDS material for delivering high molecular-weight medicines or genes into the blood stream through the intestine (Lehr, C.-M. *J. Controlled Release* 65:19-29 (2000)). However, results from research into an intestinal absorption-controlled liposome, using a sugar chain as the ligand, have not been reported. We have filed a patent application of a sugar-modified liposome wherein a sugar chain is bound to the liposome through a linker protein, the sugar chain is selected from Lewis X trisaccharide, sialyl Lewis X tetrasaccharide, 3'-sialyllactosamine trisaccharide, and 6'-sialyllactosamine trisaccharide, tris (hydroxymethyl) aminomethane is optionally bound to the liposome membrane and/or linker protein and hydrophilized, and an intestinal absorption-controlled liposome wherein the liposome is modified with a sugar chain selected from lactose disaccharide, 2'-fucosyllactose trisaccharide, difucosyllactose tetrasaccharide and 3-fucosyllactose trisaccharide and the sugar chian may be bound to the liposome through a linker protein.

However, a drug delivery system for administering therapeutic or diagnostic drugs for inflammatory diseases in a targeting manner has not been developed.

Among inflammatory diseases, rheumatoid arthritis (RA) is an autoimmune disease that accompanies various advanced symptoms primarily including the inflammation of joints, and patients with this RA are said to be approximately 0.7 million people in Japan. These patients with the RA account for the highest percentage of all patients with autoimmune diseases. The improved medical treatment of RA will make a significant contribution to society.

While RA onset is considered to be associated with genetic factors (such as particular HLA) or environmental factors (such as virus infection), the details of a mechanism underlying its onset are unknown. However, macrophages as well as CD4+ and CD8+ T cells, residing in the articular synovium have been confirmed to be present in the inflammatory site, and this T cell has been shown to recognize antigens present in the synovium (T. Toyosaki, et al., 1998 Arthritis Rheum. 41, 92-100; and P.L. van Lent, et al., 1996 Arthritis Rheum. 39, 1545-1555). Furthermore, it has bee supposed that these cells arising from inflammation are closely related to so-called inflammatory cytokines such as IL-1, tumor necrotic factor-alpha (hereinafter, referred to as TNF-a), and IL-6 (S. Saijo, et al., 2002 Arthritis Rheum. 46, 533-544; D. Kontoyiannis, et al., 1999 Immunity 10, 387-398; and T. Ohtani, et al., 2000 Immunity 12, 95-105).

These novel findings led to a major change in the recent medical treatment of RA, and its greatest progress was the development of biological preparations. Namely, a variety of therapeutic antibody drugs have been developed for TNF-alpha (hereinafter, referred to as TNF-a). These novel therapeutic antibody drugs are unique, unlike conventional therapeutic drugs, in that they have high therapeutic effects on arthritis and also possess inhibiting effects on damage to joint tissues. Meanwhile, the possibility that these novel drugs bring about serious harmful events including infectious disease and other side effects has been reported. One of causes for these side effects may be traceable to the nonspecific and systemic administration of anti-TNF-a antibodies in large quantities even for controlling local inflammatory sites such as joints.

Ideally, conventionally used drugs and even revolutionary novel therapeutic drugs, if having side effects, should be distributed specifically to lesions and should exhibit their efficacy only in the lesions. Because such a system can reduce the side effects of drugs and further enhance drug efficacy, the development of this system is also important, in addition to the development of noteworthy biological preparations and molecular target drugs currently regarded as the latest drugs.

### Disclosure of the Invention

Therefore, an object of the present invention is to provide a targeting drug delivery system (DDS) nanoparticle that can be accumulated in target tissues such as inflammatory lesions and can thus be utilized in a therapeutic or diagnostic DDS for locally delivering drugs or genes to the affected parts. More specifically, the present invention provides a targeting drug delivery system liposome in which a sugar chain capable of being bound to E-selectin, P-selectin, or the like expressed in the vascular endothelial cells of inflammatory sites is bound to its surface.

The present inventors have previously developed a targeting DDS liposome having a particular sugar chain bound to its surface. The targeting liposome has a sialyl Lewis X sugar chain (sLeX) bound to its membrane surface and possesses highly efficient mobility toward tumor sites and inflammatory sites.

The present inventors have conducted diligent studies on the application of this targeting liposome to inflammatory lesions, and have consequently completed the present invention by creating a model mouse with eye inflammation as a model animal of inflammatory disease to find out that the targeting liposome is taken into the inflammatory site of the eye in a targeting manner.

The drug delivery system (hereinafter, referred to as DDS) developed this time achieves a therapeutic system with high efficiency and fewer side effects that specifically accumulates drugs to inflammatory sites. The present inventors have used steroid drugs in Examples because they are most common antiinflammatory drugs. However, therapeutic drugs that can be used in the present invention are not limited to the steroid drugs, and the drug delivery system of the present invention allows immunosuppressive drugs, cytokines, anti-cytokine agents, and nucleic acids (including DNA and RNA) to be delivered to target sites.

That is, the present invention is as follows:
1. A pharmaceutical composition for the medical treatment or diagnosis of an inflammatory disease comprising a sugar-modified liposome having a sugar chain bound to the membrane of the liposome.
2. The pharmaceutical composition for the medical treatment or diagnosis of an inflammatory disease according to 1, wherein lipids constituting the liposome comprise phosphatidylcholines (at a molar ratio of 0 to 70%), phosphatidylethanolamines (at a molar ratio of 0 to 30%), one or more lipids (at a molar ratio of 0 to 30%) selected from the group consisting of phosphatidic acids, long-chain alkyl phosphates, and dicetylphosphoric acids, one or more lipids (at a molar ratio of 0 to 40%) selected from the group consisting of gangliosides, glycolipids, phosphatidylglycerols, and sphingomyelins, and cholesterols (at a molar ratio of 0 to 70%).
3. The pharmaceutical composition for the medical treatment or diagnosis of an inflammatory disease according to 2, wherein at least one lipid selected from the group consisting of gangliosides, glycolipids, phosphatidylglycerols, sphingomyelins, and cholesterols assembles to form a raft on the surface of the liposome.
4. The pharmaceutical composition for the medical treatment or diagnosis of an inflammatory disease according to any one of 1 to 3, wherein the sugar chain is bound to the membrane of the liposome in a manner that controls the type and density of the sugar chain.
5. The pharmaceutical composition for the medical treatment or diagnosis of an inflammatory disease according to any one of 1 to 4, wherein the liposome has a particle size of 30 to 500 nm.
6. The pharmaceutical composition for the medical treatment or diagnosis of an inflammatory disease according to 5, wherein the liposome has a particle size of 50 to 300 nm.
7. The pharmaceutical composition for the medical treatment or diagnosis of an inflammatory disease according to any one of 1 to 6, wherein the liposome has a zeta potential of -50 to 10 mV.
8. The pharmaceutical composition for the medical treatment or diagnosis of an inflammatory disease according to 7, wherein the liposome has a zeta potential of -40 to 0 mV.
9. The pharmaceutical composition for the medical treatment or diagnosis of an inflammatory disease according to 8, wherein the liposome has a zeta potential of -30 to -10 mV.
10. The pharmaceutical composition for the medical treatment or diagnosis of an inflammatory disease according to any one of 1 to 9, wherein the sugar chain is bound to the membrane of the liposome through a linker protein.
11. The pharmaceutical composition for the medical treatment or diagnosis of an inflammatory disease according to 10, wherein the linker protein is an organism-derived protein.
12. The pharmaceutical composition for the medical treatment or diagnosis of an inflammatory disease according to 11, wherein the linker protein is a human-derived protein.
13. The pharmaceutical composition for the medical treatment or diagnosis of an inflammatory disease according to 12, wherein the linker protein is a human-derived serum protein.
14. The pharmaceutical composition for the medical treatment or diagnosis of an inflammatory disease according to 10, wherein the linker protein is human serum albumin or bovine serum albumin:
15. The pharmaceutical composition for the medical treatment or diagnosis of an inflammatory disease according to any one of 1 to 14, wherein the linker protein is bound to the raft consisting of at least one lipid selected from the group consisting of gangliosides, glycolipids, phosphatidylglycerols, sphingomyelins, and cholesterols, formed on the surface of the liposome.
16. The pharmaceutical composition for the medical treatment or diagnosis of an inflammatory disease according to any one of 1 to 15, wherein the pharmaceutical composition is hydrophilized by bonding a hydrophilic compound to the membrane of the liposome and/or the linker protein.
17. The pharmaceutical composition for the medical treatment or diagnosis of an inflammatory disease according to 16, wherein the hydrophilic compound is a substance having a low molecular weight.
18. The pharmaceutical composition for the medical treatment or diagnosis of an inflammatory disease according to 16 or 17, wherein the hydrophilic compound is less apt to sterically hinder the sugar chain and does not prevent lectin on the membrane surface of a target cell from proceeding reaction of recognizing the sugar-chain molecule.
19. The pharmaceutical composition for the medical treatment or diagnosis of an inflammatory disease according to any one of 16 to 18, wherein the hydrophilic compound has a hydroxyl group.
20. The pharmaceutical composition for the medical treatment or diagnosis of an inflammatory disease according to any one of 16 to 19, wherein the hydrophilic compound is any of amino alcohols.
21. The pharmaceutical composition for the medical treatment or diagnosis of an inflammatory disease according to any one of 16 to 20, wherein the hydrophilic compound is directly bonded to the membrane surface of the liposome.
22. The pharmaceutical composition for the medical treatment or diagnosis of an inflammatory disease according to 16 which is a sugar-modified liposome, wherein the pharmaceutical composition is hydrophilized with a hydrophilic compound represented by the general formula (1):

   X-R₁(R₂OH)ₙ (1)

   wherein R₁ represents a C₁₋₄₀ linear or branched hydrocarbon chain; R₂ is absent or represents a C₁₋₄₀ linear or branched hydrocarbon chain; X represents a reactive functional group bonded either directly to the lipid of the liposome or the linker protein or to a bivalent crosslinking reagent; and n represents a natural number.
23. The pharmaceutical composition for the medical treatment or diagnosis of an inflammatory disease according to 16 which is a sugar-modified liposome, wherein the pharmaceutical composition is hydrophilized with a hydrophilic compound represented by the general formula (2):

   H₂N-R₃-(R₄OH)ₙ (2)

   wherein R₃ represents a C₁₋₄₀ linear or branched hydrocarbon chain; R₄ is absent or represents a C₁₋₄₀ linear or branched hydrocarbon chain; H₂N represents a reactive functional group bonded either directly to the lipid of the liposome or the linker protein or to a bivalent crosslinking reagent; and n represents a natural number.
24. The pharmaceutical composition for the medical treatment or diagnosis of an inflammatory disease according to 16 which is a sugar-modified liposome, wherein the pharmaceutical composition is hydrophilized with a hydrophilic compound represented by the general formula (3):

   H₂N-R₅(OH)ₙ (3)

   wherein R₅ represents a C₁₋₄₀ linear or branched hydrocarbon chain; H₂N represents a reactive functional group bonded either directly to the lipid of the liposome or the linker protein or to a bivalent crosslinking reagent; and n represents a natural number.
25. The pharmaceutical composition for the medical treatment or diagnosis of an inflammatory disease according to 16, wherein the membrane of the liposome and/or the linker protein are hydrophilized by covalently bonding a hydrophilic compound that is tris(hydroxyalkyl)aminoalkane to the membrane of the liposome and/or the linker protein.
26. The pharmaceutical composition for the medical treatment or diagnosis of an inflammatory disease according to 16, wherein the membrane of the liposome and/or the linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, and tris(hydroxypropyl)aminopropane to the membrane of the liposome and/or the linker protein.
27. The pharmaceutical composition for the medical treatment or diagnosis of an inflammatory disease according to any one of 1 to 26, wherein the sugar-modified liposome targets lectin selected from the group consisting of C-type lectin comprising selectin, DC-SIGN, DC-SGNR, collectin, and mannose-binding lectin, I-type lectin comprising siglec, P-type lectin comprising a mannose-6-phosphate receptor, R-type lectin, L-type lectin, M-type lectin, and galectin, which serves as a receptor residing on the cell-membrane surface of each tissue.
28. The pharmaceutical composition for the medical treatment or diagnosis of an inflammatory disease according to 27, wherein the sugar-modified liposome targets selectin selected from the group consisting ofE-selectin, P-selectin, and L-selectin.
29. The pharmaceutical composition for the medical treatment or diagnosis of an inflammatory disease according to any one of 1 to 28, wherein the sugar chain is bound to the liposome at a density of 1 to 60 sugar chains per linker protein molecule bound to the liposome.
30. The pharmaceutical composition for the medical treatment or diagnosis of an inflammatory disease according to any one of 1 to 28, wherein the sugar chain is bound to the liposome at a density of 1 to 30000 sugar chains per liposome molecule in the case of using the linker protein, and at a maximum density of 1 to 500000 sugar chains per liposome molecule in the case of not using the linker protein.
31. The pharmaceutical composition for the medical treatment or diagnosis of an inflammatory disease according to any one of 1 to 30, wherein the sugar chain is selected from the group consisting of Lewis X trisaccharide, sialyl Lewis X tetrasaccharide, 3'-sialyllactosamine trisaccharide, 6'-sialyllactosamine trisaccharide, α-1,2-mannobiose disaccharide, α-1,3-mannobiose disaccharide, α-1,4-mannobiose disaccharide, α-1,6-mannobiose disaccharide, α-1,3/a-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, oligomannose-9 undecasaccharide, lactose disaccharide, 2'-fucosyllactose trisaccharide, difucosyllactose tetrasaccharide, 3-fucosyllactose trisaccharide, 3'-sialyllactose trisaccharide, and 6'-sialyllactose trisaccharide.
32. The pharmaceutical composition for the medical treatment of an inflammatory disease according to any one of 1 to 31, wherein the sugar-modified liposome comprises a drug selected from the group consisting of adrenocortical hormones, antiinflammatory drugs, immunosuppressive drugs, anticancer drugs, antimicrobial drugs, antiviral drugs, angiogenesis inhibitors, cytokines, chemokines, anti-cytokine antibodies, anti-chemokine antibodies, anti-cytokine/chemokine receptor antibodies, nucleic acid preparations for therapy using genes such as siRNA and DNA, neuroprotective factors, and antibody drugs.
33. The pharmaceutical composition for the medical treatment of an inflammatory disease according to 32, wherein the sugar-modified liposome comprises an adrenocortical hormone or an antiinflammatory drug as the drug.
34. The pharmaceutical composition for the medical treatment of an inflammatory disease according to 33, wherein the sugar-modified liposome comprises prednisolone as the drug.
35. The pharmaceutical composition for the medical treatment of an inflammatory disease according to any one of 32 to 34, wherein the drug can be accumulated in an inflammatory site at a level 10 or more times higher than that of the drug administered alone.
36. The pharmaceutical composition for the medical treatment or diagnosis of an inflammatory disease according to any one of 1 to 35, wherein the inflammatory disease is selected from the group consisting of encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases such as rheumatism, systemic lupus erythematosus, and sarcoidosis, ischemic diseases such as myocardial infarction and cerebral infarction, metabolic disease such as diabetes and gout, injury, scald, chemical corrosion, and neurodegenerative diseases such as Alzheimer's disease.
37. The pharmaceutical composition for the medical treatment or diagnosis of an inflammatory disease according to 36, wherein the inflammatory disease is inflammatory eye disease.
38. The pharmaceutical composition for the medical treatment or diagnosis of an inflammatory disease according to 36, wherein the inflammatory disease is rheumatism.
39. The pharmaceutical composition for the medical treatment or diagnosis of an inflammatory disease according to 36, wherein the inflammatory disease is enteritis.
40. The pharmaceutical composition for the medical treatment or diagnosis of an inflammatory disease according to any one of 1 to 39, wherein the pharmaceutical composition is a pharmaceutical composition for oral administration.
41. The pharmaceutical composition for the medical treatment or diagnosis of an inflammatory disease according to any one of 1 to 39, wherein the pharmaceutical composition is a pharmaceutical composition for parenteral administration.
42. A pharmaceutical composition for the medical treatment or diagnosis of an inflammatory disease comprising a liposome having a hydrophilized membrane surface to which no sugar chain is bound.
43. The pharmaceutical composition for the medical treatment or diagnosis of an inflammatory disease according to 42, wherein lipids constituting the liposome comprise phosphatidylcholines (at a molar ratio of 0 to 70%), phosphatidylethanolamines (at a molar ratio of 0 to 30%), one or more lipids (at a molar ratio of 0 to 30%) selected from the group consisting of phosphatidic acids, long-chain alkyl phosphates, and dicetylphosphoric acids, one or more lipids (at a molar ratio of 0 to 40%) selected from the group consisting of gangliosides, glycolipids, phosphatidylglycerols, and sphingomyelins, and cholesterols (at a molar ratio of 0 to 70%).
44. The pharmaceutical composition for the medical treatment or diagnosis of an inflammatory disease according to 43, wherein the liposome further comprises a protein.
45. The pharmaceutical composition for the medical treatment or diagnosis of an inflammatory disease according to any one of 42 to 44, wherein the pharmaceutical composition is hydrophilized by bonding a hydrophilic compound to the membrane of the liposome and/or a linker protein.
46. The pharmaceutical composition for the medical treatment or diagnosis of an inflammatory disease according to 45, wherein the hydrophilic compound is a substance having a low molecular weight.
47. The pharmaceutical composition for the medical treatment or diagnosis of an inflammatory disease according to 45 or 46, wherein the hydrophilic compound has a hydroxyl group.
48. The pharmaceutical composition for the medical treatment or diagnosis of an inflammatory disease according to any one of 45 to 47, wherein the hydrophilic compound is any of amino alcohols.
49. The pharmaceutical composition for the medical treatment or diagnosis of an inflammatory disease according to any one of 45 to 48, wherein the hydrophilic compound is directly bonded to the membrane surface of the liposome.
50. The pharmaceutical composition for the medical treatment or diagnosis of an inflammatory disease according to 45, wherein the pharmaceutical composition is hydrophilized with a hydrophilic compound represented by the general formula (1):

   X-R₁(R₂OH)ₙ (1)

   wherein R₁ represents aC₁₋₄₀ linear or branched hydrocarbon chain; R₂ is absent or represents a C₁₋₄₀ linear or branched hydrocarbon chain; X represents a reactive functional group bonded either directly to the lipid of the liposome or the linker protein or to a bivalent crosslinking reagent; and n represents a natural number.
51. The pharmaceutical composition for the medical treatment or diagnosis of an inflammatory disease according to 45, wherein the pharmaceutical composition is hydrophilized with a hydrophilic compound represented by the general formula (2):

   H₂N-R₃-(R₄OH)ₙ (2)

   wherein R₃ represents a C₁₋₄₀ linear or branched hydrocarbon chain; R₄ is absent or represents a C₁₋₄₀ linear or branched hydrocarbon chain; H₂N represents a reactive functional group bonded either directly to the lipid of the liposome or the linker protein or to a bivalent crosslinking reagent; and n represents a natural number.
52. The pharmaceutical composition for the medical treatment or diagnosis of an inflammatory disease according to 45, wherein the pharmaceutical composition is hydrophilized with a hydrophilic compound represented by the general formula (3):

   H₂N-R₅(OH)ₙ (3)

   wherein R₅ represents a C₁₋₄₀ linear or branched hydrocarbon chain; H₂N represents a reactive functional group bonded either directly to the lipid of the liposome or the linker protein or to a bivalent crosslinking reagent; and n represents a natural number.
53. The pharmaceutical composition for the medical treatment or diagnosis of an inflammatory disease according to 45, wherein the membrane of the liposome and/or the linker protein are hydrophilized by covalently bonding a hydrophilic compound that is tris(hydroxyalkyl)aminoalkane to the membrane of the liposome and/or the linker protein.
54. The pharmaceutical composition for the medical treatment or diagnosis of an inflammatory disease according to 53, wherein the membrane of the liposome and/or the linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, and tris(hydroxypropyl)aminopropane to the membrane of the liposome and/or the linker protein.
55. The pharmaceutical composition for the medical treatment of an inflammatory disease according to any one of 42 to 54, wherein the liposome comprises a drug selected from the group consisting of adrenocortical hormones, antiinflammatory drugs, immunosuppressive drugs, anticancer drugs, antimicrobial drugs, antiviral drugs, angiogenesis inhibitors, cytokines, chemokines, anti-cytokine antibodies, anti-chemokine antibodies, anti-cytokine/chemokine receptor antibodies, nucleic acid preparations for therapy using genes such as siRNA and DNA, neuroprotective factors, and antibody drugs.
56. The pharmaceutical composition for the medical treatment of an inflammatory disease according to 55, wherein the liposome comprises an adrenocortical hormone or an antiinflammatory drug as the drug.
57. The pharmaceutical composition for the medical treatment of an inflammatory disease according to 56, wherein the liposome comprises prednisolone as the drug.
58. The pharmaceutical composition for the medical treatment of an inflammatory disease according to any one of 55 to 57, wherein the drug can be accumulated in an inflammatory site at a level 10 or more times higher than that of the drug administered alone.
59. The pharmaceutical composition for the medical treatment or diagnosis of an inflammatory disease according to any one of 42 to 58, wherein the inflammatory disease is selected from the group consisting of encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases such as rheumatism, systemic lupus erythematosus, and sarcoidosis, ischemic disease such as myocardial infarction and cerebral infarction, metabolic diseases such as diabetes and gout, injury, scald, chemical corrosion, and neurodegenerative disease such as Alzheimer's disease.
60. The pharmaceutical composition for the medical treatment or diagnosis of an inflammatory disease according to 59, wherein the inflammatory disease is inflammatory eye disease.
61. The pharmaceutical composition for the medical treatment or diagnosis of an inflammatory disease according to 59, wherein the inflammatory disease is rheumatism.
62. The pharmaceutical composition for the medical treatment or diagnosis of an inflammatory disease according to 59, wherein the inflammatory disease is enteritis.
63. The pharmaceutical composition for the medical treatment or diagnosis of an inflammatory disease according to any one of 42 to 62, wherein the pharmaceutical composition is a pharmaceutical composition for oral administration.
63. The pharmaceutical composition for the medical treatment or diagnosis of an inflammatory disease according to any one of 42 to 62, wherein the pharmaceutical composition is a pharmaceutical composition for parenteral administration.

The present specification encompasses contents described in the specifications and/or drawings of Japanese Patent Application Nos. 2003-285422 and 2003-369494 that serve as a basis for the priority of the present application.

### Brief Description of the Drawings

Fig. 1 is a schematic diagram illustrating a structure of a liposome modified by Lewis X trisaccharide is bound;
Fig. 2 is a schematic diagram illustrating a structure of a liposome modified by sialyl Lewis X tetrasaccharide is bound;
Fig. 3 is a schematic diagram illustrating a structure of a liposome modified by 3'-sialyllactosamine trisaccharide is bound;
Fig. 4 is a schematic diagram illustrating a structure of a liposome modified by 6'-sialyllactosamine trisaccharide is bound;
Fig. 5 is a schematic diagram illustrating a structure of a liposome modified by α-1,2-mannobiose disaccharide is bound;
Fig. 6 is a schematic diagram illustrating a structure of a liposome modified by α-1,3-mannobiose disaccharide is bound;
Fig. 7 is a schematic diagram illustrating a structure of a liposome modified by α-1,4-mannobiose disaccharide is bound;
Fig. 8 is a schematic diagram illustrating a structure of a liposome modified by α-1,6-mannobiose disaccharide is bound;
Fig. 9 is a schematic diagram illustrating a structure of a liposome modified by α-1,3/α-1,6-mannotriose trisaccharide is bound;
Fig. 10 is a schematic diagram illustrating a structure of a liposome modified by oligomannose-3 pentasaccharide is bound;
Fig. 11 is a schematic diagram illustrating a structure of a liposome modified by oligomannose-4b hexasaccharide is bound;
Fig. 12 is a schematic diagram illustrating a structure of a liposome modified by oligomannose-5 heptasaccharide is bound;
Fig. 13 is a schematic diagram illustrating a structure of a liposome modified by oligomannose-6 octasaccharide is bound;
Fig. 14 is a schematic diagram illustrating a structure of a liposome modified by oligomannose-7 nonasaccharide is bound;
Fig. 15 is a schematic diagram illustrating a structure of a liposome modified by oligomannose-8 decasaccharide is bound;
Fig. 16 is a schematic diagram illustrating a structure of a liposome modified by oligomannose-9 undecasaccharide is bound;
Fig. 17 is a schematic diagram illustrating a structure of a liposome modified by lactose disaccharide;
Fig. 18 is a schematic diagram illustrating a structure of a liposome modified by 2'-fucosyllactose trisaccharide;
Fig. 19 is a schematic diagram illustrating a structure of a liposome modified by difucosyllactose tetrasaccharide;
Fig. 20 is a schematic diagram illustrating a structure of a liposome modified by 3-fucosyllactose trisaccharide;
Fig. 21 is a schematic diagram illustrating a structure of a liposome modified by 3'-sialyllactose trisaccharide is bound;
Fig. 22 is a schematic diagram illustrating a structure of a liposome modified by 6'-sialyllactose trisaccharide is bound;

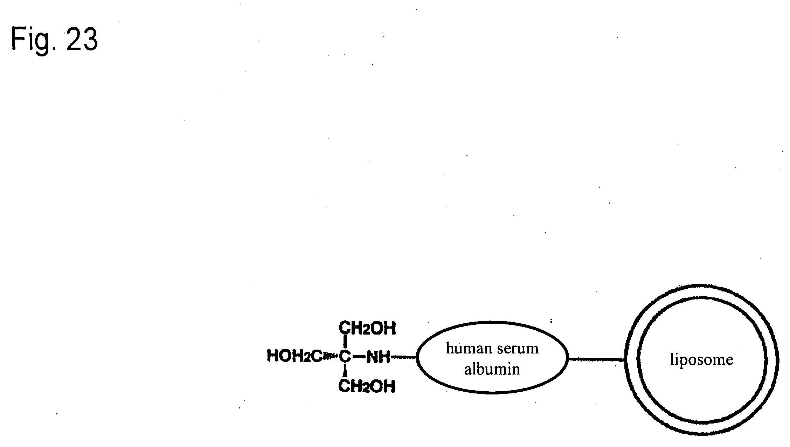

Fig. 23 is a schematic diagram of a liposome to which tris(hydroxymethyl)aminomethane, as a comparative sample;
Fig. 24 is a photograph showing experimental uveitis onset;
Fig. 25A is a diagram showing the distribution of liposomes in different organs;
Fig. 25B is a diagram showing the distribution of liposomes in different organs, which is given in % relative value compared to in normal mice;
Fig. 26 is a photograph showing a temporal shift in the distribution of a liposome;
Fig. 27 is a photograph showing the distribution of liposomes in normal mice and in EAU mice;
Fig. 28 is a photograph showing a result of detection through a fluorescent antibody method;
Fig. 29 is a photograph showing a result of detection through an enzyme antibody method;
Fig. 30 is a photograph showing a result of a binding inhibition test;
Fig. 31 is a photograph showing the inflamed limbs ofRA mice;
Fig. 32 is a diagram showing a result of treating RA mice by the intravenous administration of a DDS liposome incorporating prednisolone therein;
Fig. 33 is a diagram showing a result of treating RA mice by the oral administration of a DDS liposome incorporating prednisolone therein;
Fig. 34 is a diagram showing a result of treating RA mice by the administration of a proper amount of prednisolone; and
Fig. 35 is a diagram showing a result of comparison of sustainability in blood among 2 hydrophilized liposomes and an unhydrophilized liposome after 5 minutes from their administration into the tail veins of mice with cancer.

### Best Mode for Carrying Out the Invention

The present invention provides a targeting liposome having a targeting performance to the lesions of inflammatory diseases and capable of being specifically taken into the lesions to release an encapsulated drug in the lesions, thereby treating the lesions.

It is known that E-selectin and P-selectin expressed in vascular endothelial cells during inflammation are strongly bound to a sialyl Lewis X sugar chain expressed on the cell membrane of leukocytes. The liposome of the present invention is a liposome in which this sialyl Lewis X sugar chain or a sugar chain that can be reacted with E-selectin, P-selectin, or the like as in the sialyl Lewis X sugar chain is bound to its membrane in a manner that controls the type and density of the sugar chain. This liposome is considered to be specifically accumulated in lesions having vascular endothelial cells with the expression of E-selectin, P-selectin, or the like. Moreover, inflammation and angiogenesis also occur in sites having the expression of E-selectin, P-selectin, or the like, and the blood vessels of such sites have the expanded gaps between endothelial cells, from which the accumulated liposome will be diffused to the lesions and their surroundings. The diffused liposome is taken into the lesions and a variety of their neighboring cells (phagocytosis) to release an encapsulated drug in the cells. Based on such a mechanism, the liposome of the present invention exhibits its effects on inflammatory diseases.

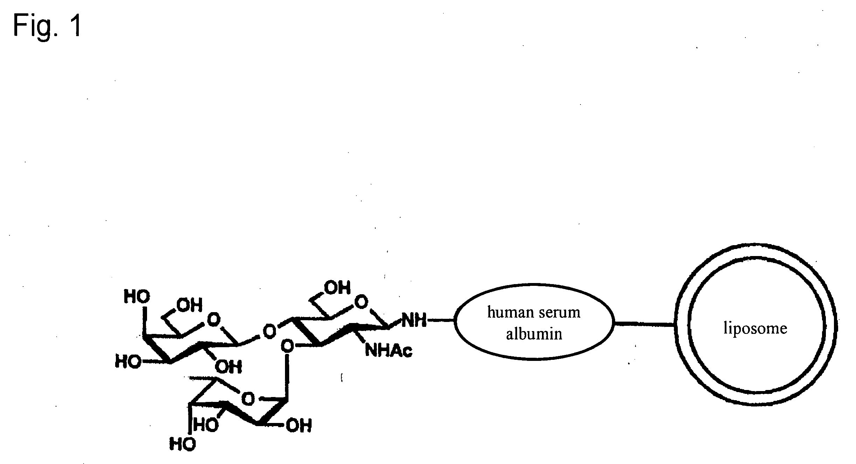

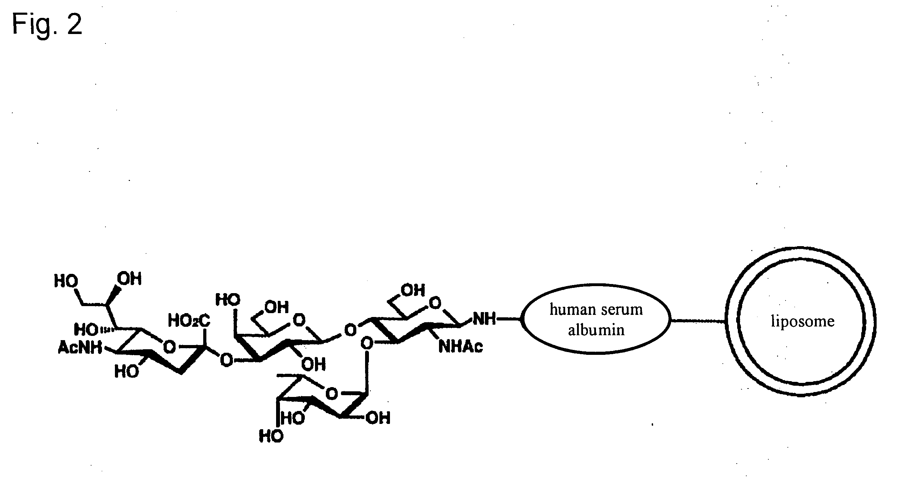

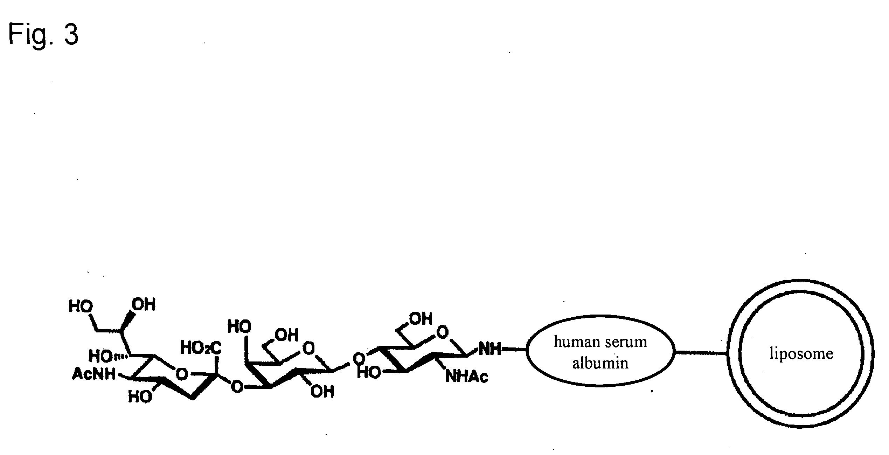

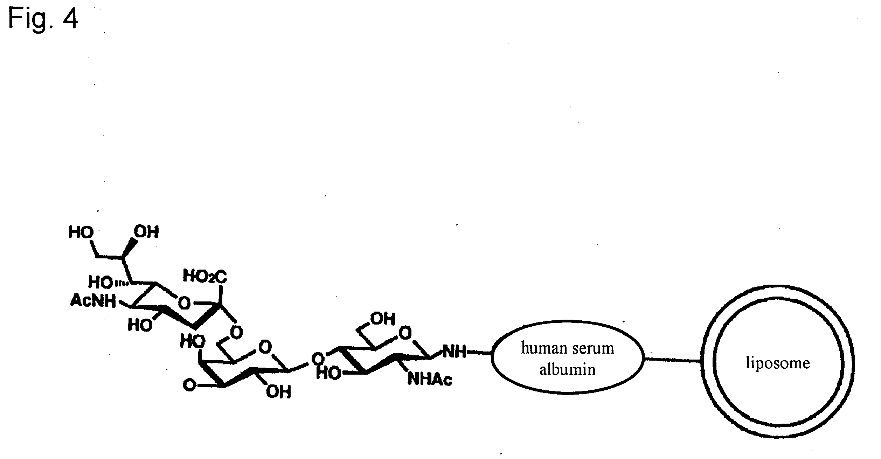

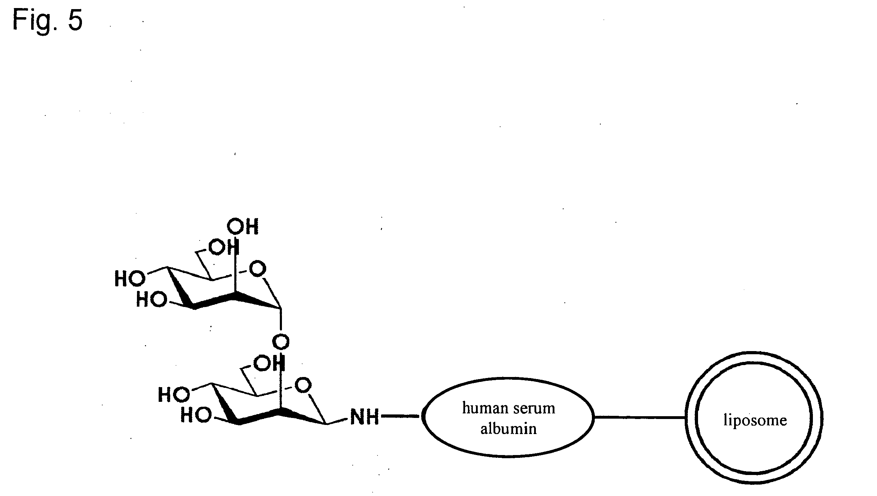

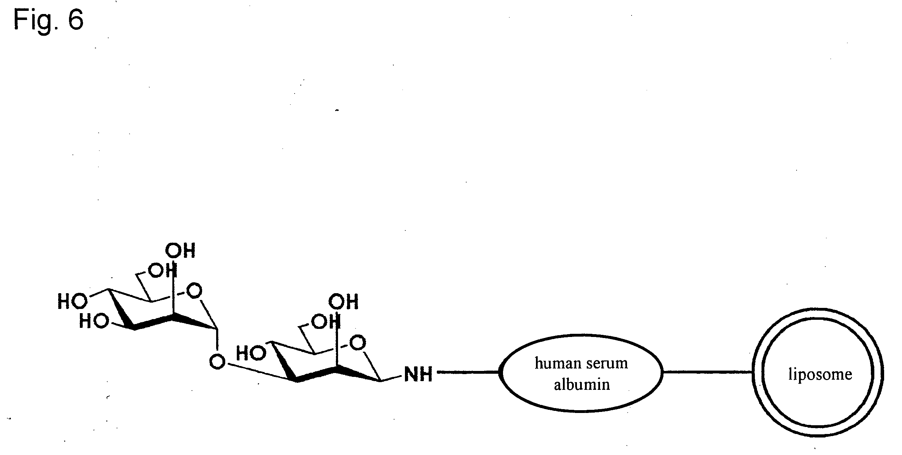

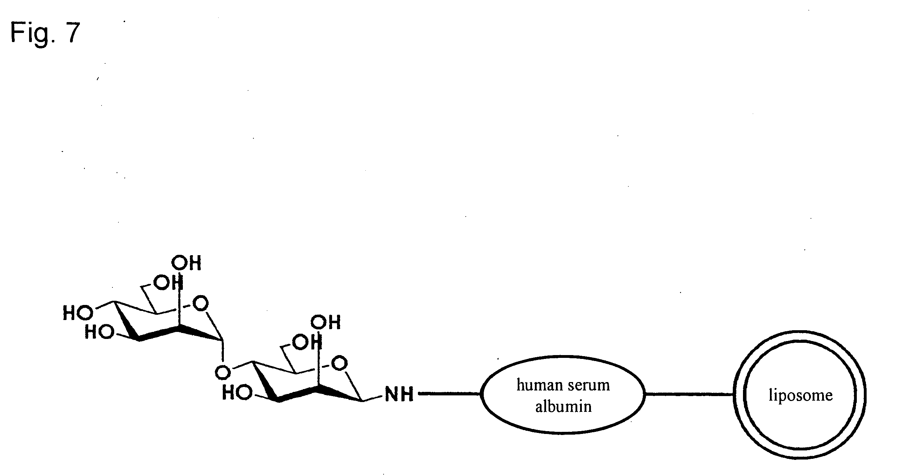

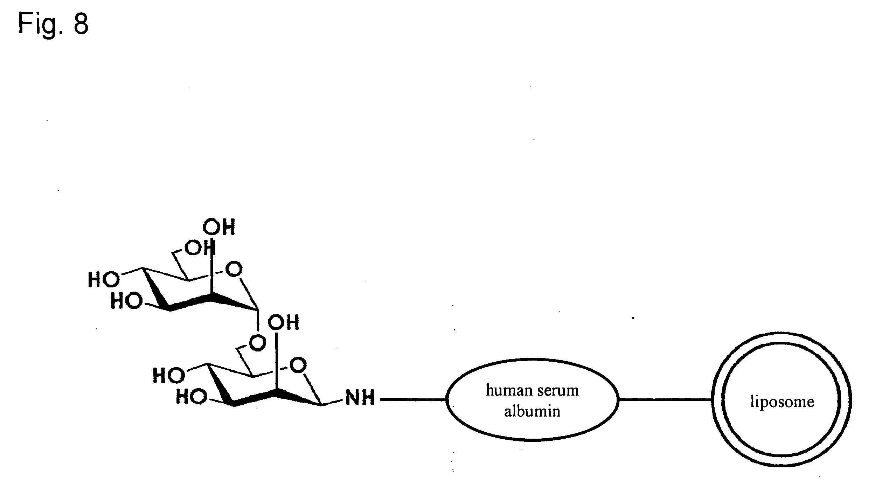

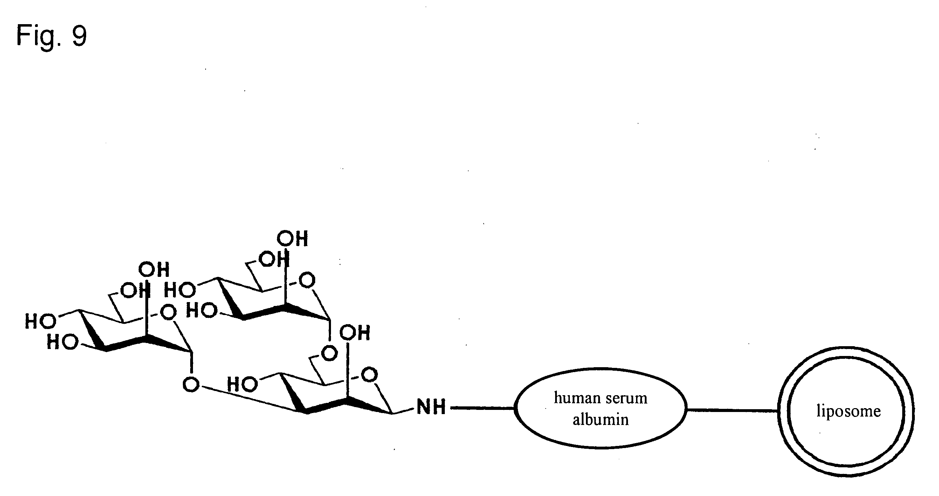

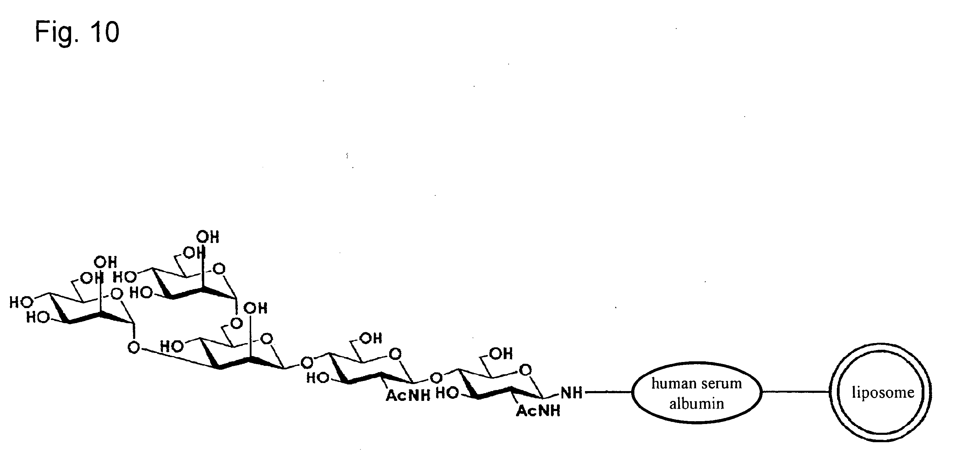

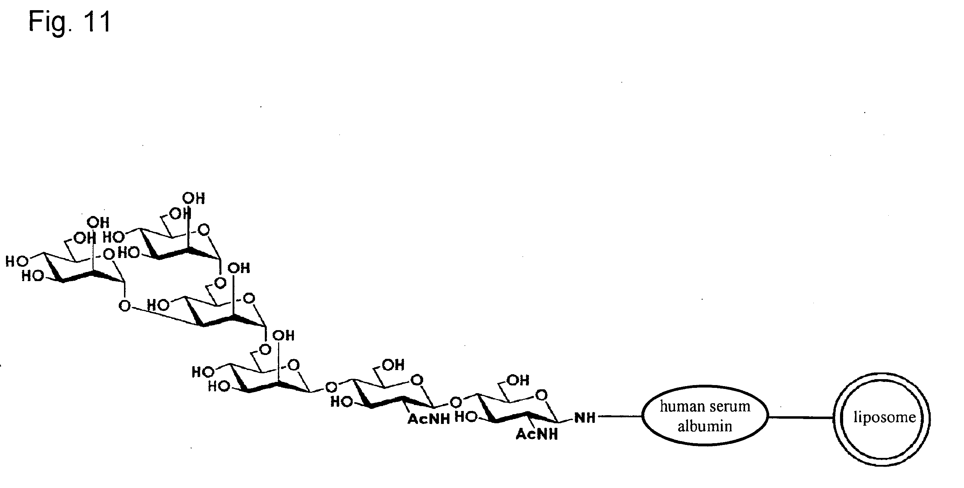

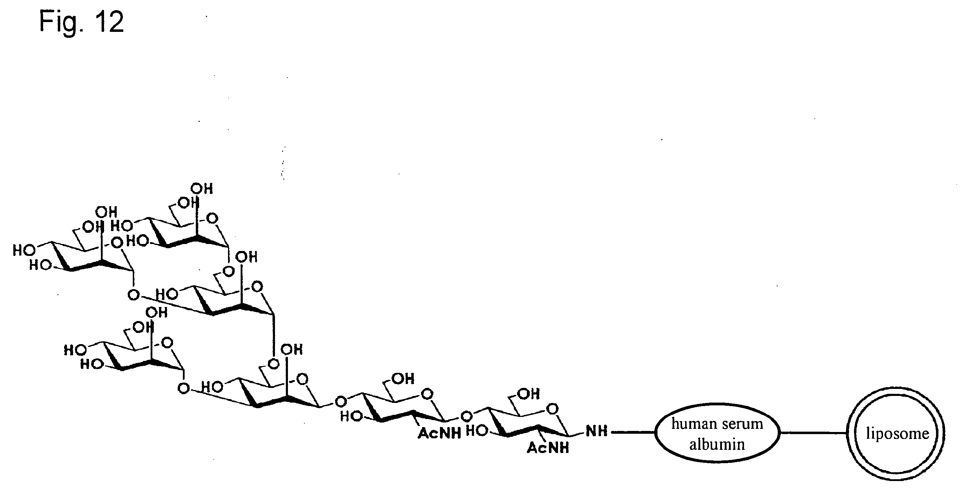

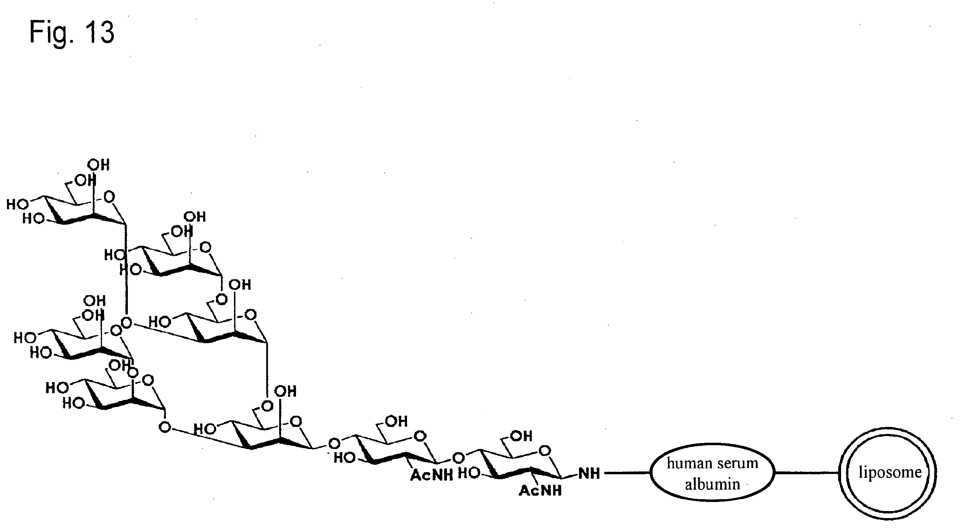

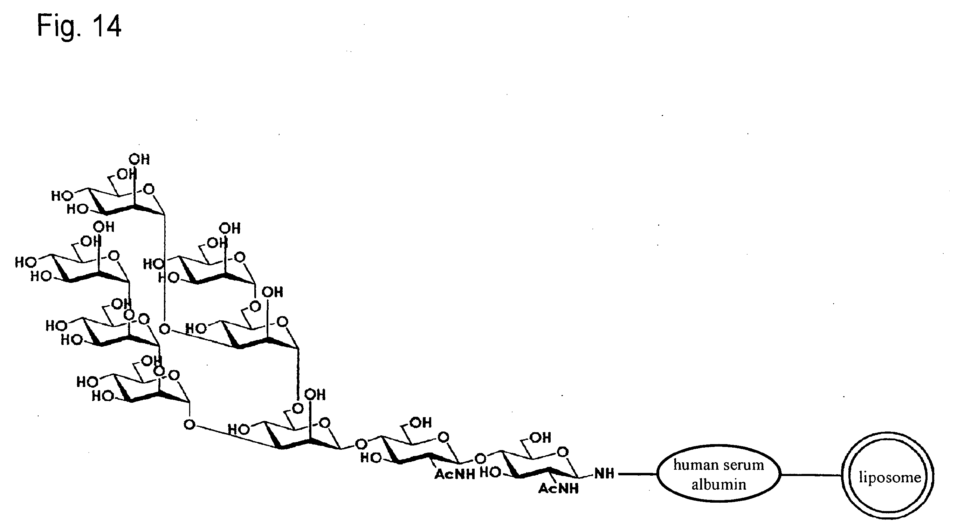

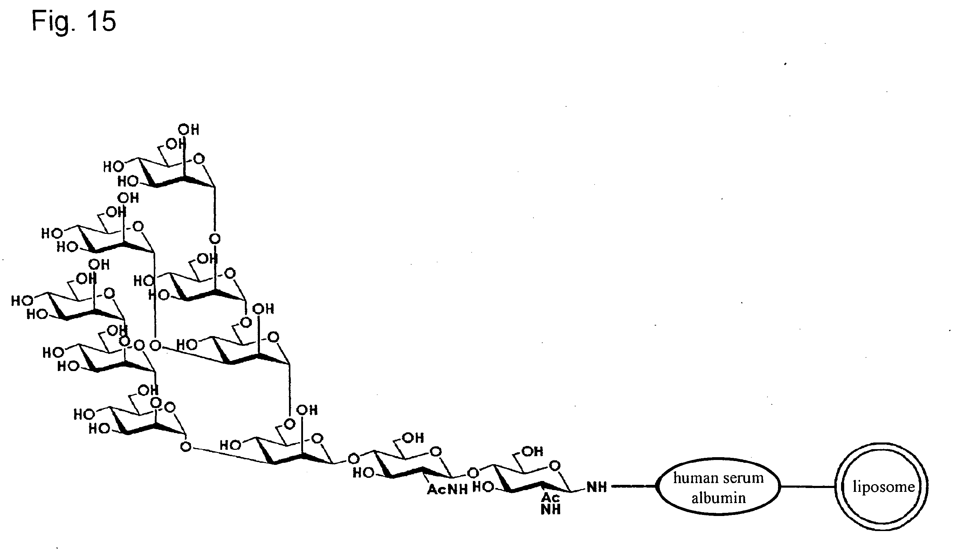

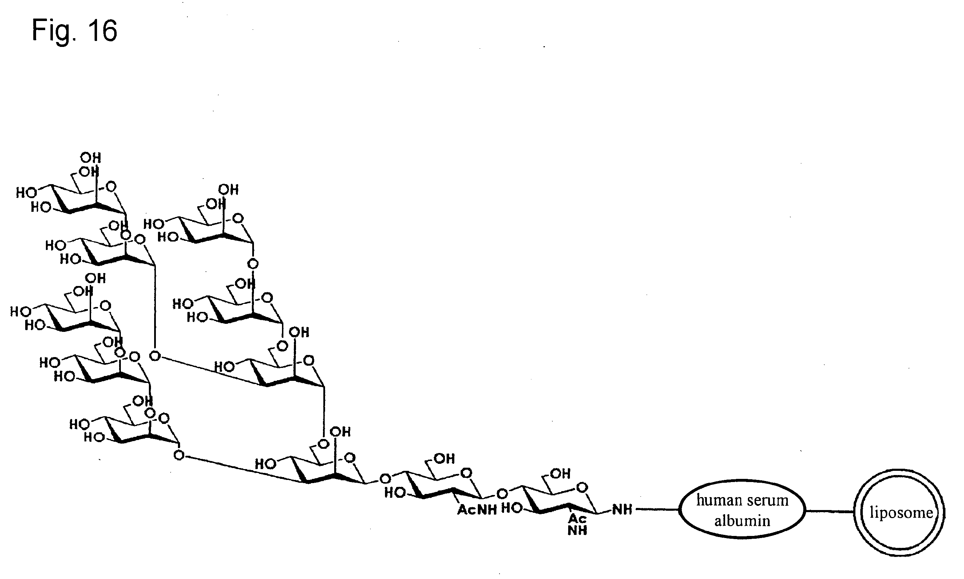

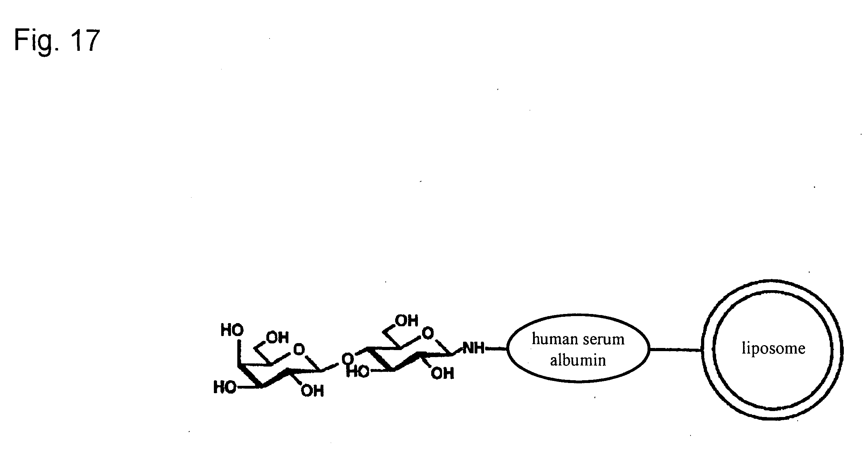

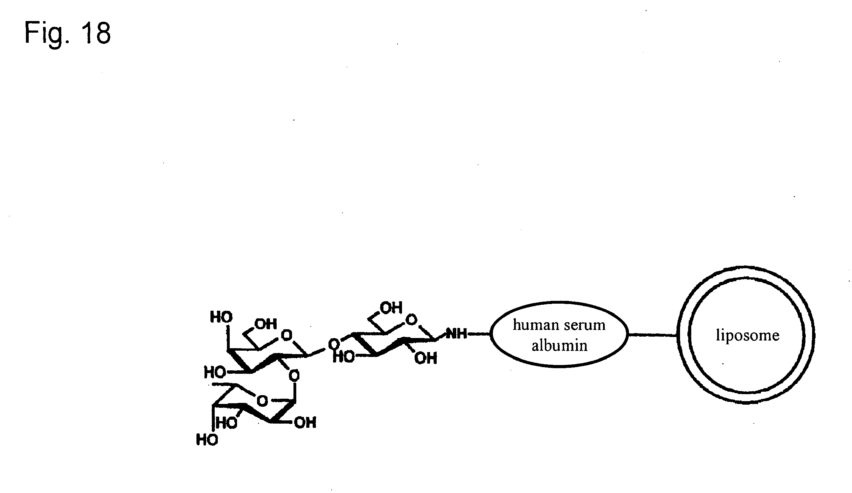

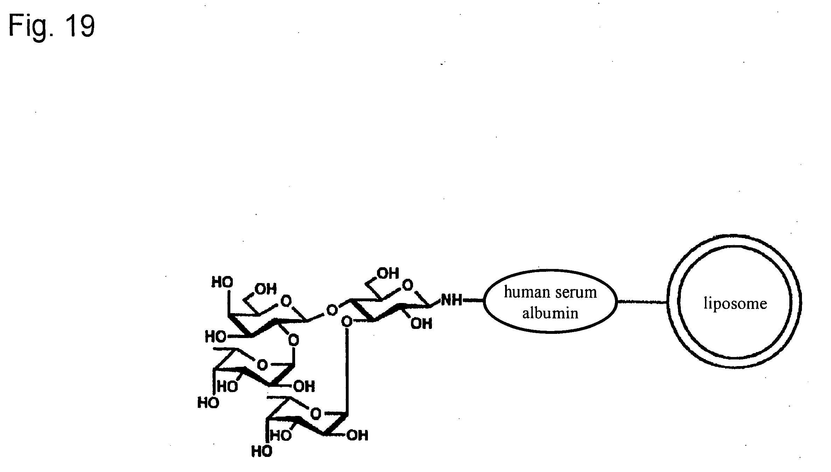

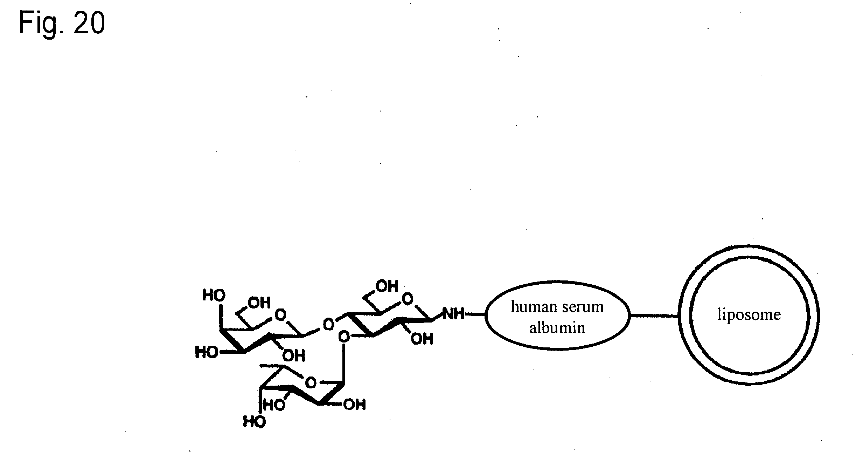

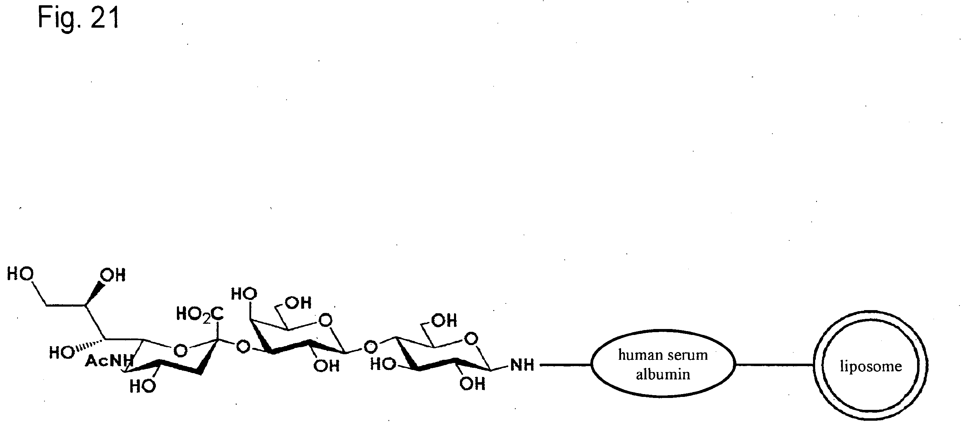

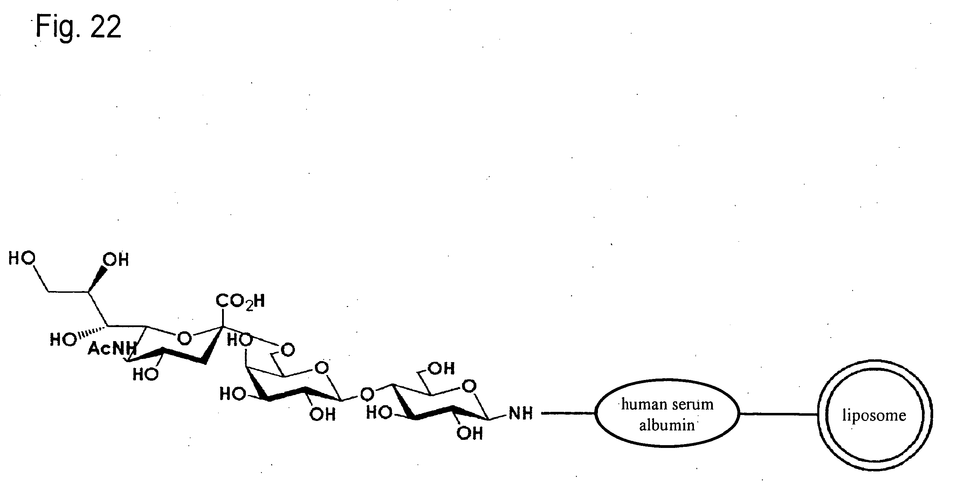

As the sugar chain to be bonded to the liposome of the present invention, mention may be made of a sugar chain reactive to a protein, such as E-selectin and P-selectin, having a sugar chain binding site as mentioned above. The E-selectin and P-selectin herein refer to various types of lectins (sugar-chain recognizing proteins), which include C-type lectins such as selectin, DC-SIGN, DC-SGNR, colectin, mannose binding protein, I-type lectins such as siglec, P-type lectins such as a mannose-6-phosphate receptor, R-type lectin, L-type lectin, M-type lectin, and galectin. Examples of such a sugar chain include, but not limited to, Lewis X trisaccharide (its structural formula is shown in Fig. 1; hereinafter, only the drawing number is described), sialyl Lewis X tetrasaccharide (Fig. 2), 3'-sialyllactosamine trisaccharide (Fig. 3), 6'-sialyllactosamine trisaccharide (Fig. 4), α-1,2-mannobiose disaccharide (Fig. 5), α-1,3-mannobiose disaccharide (Fig. 6), α-1,4-mannobiose disaccharide (Fig. 7), α-1,6-mannobiose disaccharide (Fig. 8), α-1,3/a-1,6-mannotriose trisaccharide (Fig. 9), oligomannose-3 pentasaccharide (Fig. 10), oligomannose-4b hexasaccharide (Fig. 11), oligomannose-5 heptasaccharide (Fig. 12), oligomannose-6 octasaccharide (Fig. 13), oligomannose-7 nonasaccharide (Fig. 14), oligomannose-8 decasaccharide (Fig. 15), oligomannose-9 undecasaccharide (Fig. 16), lactose disaccharide (Fig. 17), 2'-fucosyllactose trisaccharide (Fig. 18), difucosyllactose tetrasaccharide (Fig. 19), 3-fucosyllactose trisaccharide (Fig. 20), 3'-sialyllactose trisaccharide (Fig. 21), and 6'-sialyllactose trisaccharide (Fig. 22).

### (1) Production of targeting liposomes

The "liposome" generally refers to a closed vesicle composed of a lipid layer, which is a membrane-form lipid assembly and an aqueous layer formed in the lipid layer. The liposome of the present invention has sugar chains bonded onto surface, that is, the lipid layer, as shown in Figures 1 to 2. The sugar chain may be bonded to the lipid layer of a liposome directly or covalently via a linker protein such as human serum albumin.
Examples of the lipids constituting the liposome of the present invention include phosphatidylcholines, phosphatidylethanolamines, phosphatidic acids, long-chain alkyl phosphates, gangliosides, glycolipids, phosphatidylglycerols, sphingomyelins, and cholesterols. Examples of the phosphatidylcholines preferably include dimyristoylphosphatidylcholine, dipalmitoylphosphatidylcholine, and distearoylphosphatidylcholine. Examples of phosphatidylethanolamines preferably include dimyristoylphosphatidyl ethanolamine, dipalmitoylphosphatidylethanolamine, and distearoyl phosphatidylethanolamine. Examples of the phosphatidic acids or long-chain alkyl phosphates preferably include dimyristoylphosphatidic acid, dipalmitoylphosphatidic acid, distearoylphosphatidic acid, and dicetylphosphoric acid. Examples of the gangliosides preferably include ganglioside GM1, ganglioside GD1 a, and ganglioside GT1 b. Examples of glycolipids preferably include galactosylceramide, glycosylceramide, lactosylseramide, phosphatide, and globoside. Examples of the phosphatidylglycerols preferably include dimyristoylphosphatidylglycerol, dipalmitoylphosphatidylglycerol, and distearoylphosphatidylglycerol. Of them, a phosphatidic acid, a long-chain alkyl phosphate, ganglioside, glycolipid, or cholesterol is desirably added as a constitutional lipid since they have an effect of increasing stability of a liposome.
Examples of the lipid constituting the liposome of the present invention include phosphatidylcholines (at a molar ratio of 0 to 70%), phosphatidylethanolamines (at a molar ratio of 0 to 30%), one or more lipids (at a molar ratio of 0 to 30%) selected from the group consisting of phosphatidic acids, long-chain alkyl phosphates, and dicetylphosphoric acids, one or more lipids (at a molar ratio of 0 to 40%) selected from the group consisting of gangliosides, glycolipids, phosphatidylglycerols, and sphingomyelins, and cholesterols (at a molar ratio of 0 to 70%).

The liposome itself can be produced in accordance with a customary method. Examples of such a customary method include a thin-film method, reverse layer vaporization method, ethanol injection method, and dehydration-rehydration method.

Furthermore, the particle diameter of a liposome may be controlled by an ultrasonic irradiation method, extrusion method, French press method, or homogenization method. A method of producing the liposome of the present invention will be more specifically described. First, a mixed micelle is prepared by a lipid containing components such as a phosphatidylchlorin, cholesterol, a phosphatidylethanolamine, phosphatidic acid, ganglioside, glycolipid or phosphatidylglycerol, and sodium cholate serving as a surfactant.

Particularly, the phosphatidylethanolamines must absolutely be compounded for providing a hydrophilic reaction site, and the gangliosides, glycolipids, or phosphatidylglycerols must absolutely be compounded for providing a bonding site for the linker protein. The mixed micelle thus obtained is subjected to ultrafiltration to prepare a liposome.

As the liposome to be used in the present invention, a general liposome may be used. However, the surface of the liposome is desirably hydrophilized. After a liposome is prepared as described above, the surface of the liposome is hydrophilized. The hydrophilization of the liposome surface is performed by binding a hydrophilic compound thereto. As a compound that may be used in hydrophilization, use may be made of a low molecular weight hydrophilic compound, preferably a low molecular weight hydrophilic compound having at least one OH group, more preferably, a low molecular weight hydrophilic compound having at least two OH groups. Furthermore, mention may be made of a low molecular weight hydrophilic compound having at least one amino group, in other words, a hydrophilic compound having at least one OH group and at least one amino group. Since a hydrophilic compound has a low molecular weight, it rarely causes steric hindrance to a sugar chain and thus does not prevent proceeding of a sugar-chain molecular recognition reaction by a lectin on the surface of a target cell membrane. Furthermore, examples of the hydrophilic compound do not include a sugar chain capable of binding a lectin used for targeting to a specific target such as a lectin in the liposome modified by a sugar chain of the present invention. As an examples of such a hydrophilic compound, use may be made of amino alcohols such as tris(hydroxyalkyl)aminoalkane including tris(hydroxymethyl) aminomethane, more specifically, tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane. Furthermore, a low molecular weight compound having an OH group to which an amino group is introduced may be used as a hydrophilic compound according to the present invention. Such a compound is not limited and, for example, cellobiose may be mentioned which has an amino group introduced into a sugar chain to which a lectin is not bonded. The surface of a liposome is hydrophilized by applying a divalent crosslinking agent and tris(hydroxymethyl)aminomethane onto a lipid, phosphatidylethanolamine of a liposome membrane. A hydrophilic compound is represented by the following general formulas (1), (2) and (3).

X-R1(R2OH)n Formula (1)

H₂N-R3-(R4OH)n Formula (2)

H₂N-R5(OH)n Formula (3)

In the formula, R1, R3 and R5 represent a linear or branched hydrocarbon chain of C1 to C40, preferably C1to C20, further preferably C1 to C10; and R2 and R4 are absent or represent a linear or branched hydrocarbon chain of C1 to C40, preferably C1 to C20, further preferably C1 to C 10. X represents a reactive functional group directly binding to a liposome lipid or a divalent crosslinking agent. Examples of such a functional group include COOH, NH, NH₂, CHO, SH, NHS-ester, maleimide, imidoester, active halogen, EDC, pyridyldisulfide, azidephenyl, and hydrazide. A reference symbol n represents a natural number.

The hydrophilization of a liposome may be performed by a method (JP Patent Publication (Kokai) No. 2000-302685) of producing a liposome by using a phospholipid, which is prepared by covalently bonding polyethylene glycol, polyvinyl alcohol and an anhydrous maleic acid copolymer.

Of them, hydrophilization of a liposome surface is particularly preferably performed by use of tris(hydroxymethyl)aminomethane.

The hydrophilization method using a low molecular weight hydorophilic compound such as tris(hydroxymethyl)aminomethane of the present invention is preferable compared to a conventional method using polyethylene glycol in several points. For example, in the case of the present invention where targeting is performed by use of a molecular recognition function of a sugar chain which is bonded onto a liposome, a low molecular weight hydorophilic compound such as tris(hydroxymethyl)aminomethane is particularly preferably used, since a low molecular weight hydorophilic compound such as tris(hydroxymethyl)aminomethane is a low molecular weight substance compared to a conventionally used high molecular weight substance such as polyethylene glycol, it rarely causes steric hindrance to the sugar chain, and does not prevent proceeding of a sugar-chain molecular recognition reaction performed by a lectin (sugar chain recognizing protein) on the membrane surface of a target cell.

Furthermore, the liposome of the present invention has a satisfactory size distribution, composition, and dispersion properties after completion of the hydrophilization treatment, and excellent long-term storage stability and *in-vivo* stability. Therefore, the liposome of the present invention is preferably used for producing a preparation.

To hydrophilize a liposome surface by a low molecular weight hydorophilic compound such as tris(hydroxymethyl)aminomethane, first a divalent reagent such as bissulfosuccinimidylsuberate, disuccinimidyl glutarate, dithiobissuccinimidylpropionate, disuccinimidylsuberate, 3,3'-dithiobissurfosuccinimidylpropionate, ethylene glycol bissuccinimidylsuccinate, or ethylene glycol bissulfosuccinimidylsuccinate, is added to a liposome solution, which is obtained in accordance with a customary method using a lipid such as dimyristoylphosphatidylethanolamine, dipalmitoylphosphatidyl ethanolamine, or distearoylphosphatidylethanolamine to react them, thereby bonding the divalent regent to the lipid such as dipalmitoylphosphatidyl ethanolamine on the liposome membrane; and then tris(hydroxymethyl)aminomethane is reacted with one of the bonds of the divalent reagent, thereby bonding tris(hydroxymethyl)aminomethane to the liposome surface.

The hydrophilic compound is bound to the liposome surface at a density of 1 to 500000 molecules, preferably 1 to 50000 molecules, per liposome molecule.

The liposome hydrophilized in this manner is extremely stable *in vivo.* Since it has a long *in vivo* half-life, even if a targeting sugar chain having targetability is not bonded as described later, the liposome can be preferably used as a drug carrier in the drug delivery system. The present invention includes a liposome hydrophilized with a low molecular weight compound in its surface.

The present invention includes a liposome hydrophilized with a hydrophilic compound as mentioned above and having no sugar chain bonded thereto. Such a hydrophilized liposome is advantageous since the stability of the liposome itself is high and the recognition of a sugar chain by the liposome is enhanced when a sugar chain is bonded to it.

In the present invention, any one of the sugar chains mentioned above may be directly bonded to the liposome prepared in the aforementioned manner and further bonded to the liposome via a linker protein. In this case, the type of a sugar chain to be bonded to a liposome is not limited to a single type. A plurality of types of sugar chains may be bonded. The plurality of types of sugar chains may bind to different lectins commonly present on the cellular surface of the same tissue or organ or different lectins present on the cellular surface of different tissues and organs. If the former case of sugar chain is selected, the liposome can be surely directed to a predetermined target tissue or organ. If the latter case of sugar chain is selected, a single type of liposome can be directed to a plurality of targets. In this way, multi-purpose targeting liposome can be obtained.

To bind a sugar chain to a liposome, a linker protein and/or the sugar chain are mixed in producing the liposome, thereby bonding the sugar chain on the surface of the liposome while producing the liposome. Alternatively, it is rather desirable to separately prepare a liposome, linker protein and sugar chain(s) and then bind the linker protein and/or the sugar chain to the liposome after completion of liposome production. This is because the binding density of the sugar chains can be controlled by bonding a linker protein and/or a sugar chain to the liposome.

Direct binding of a sugar chain to a liposome can be performed by the method described below.

A liposome is produced by mixing a sugar chain in the form of a glycolipid or by binding a sugar chain to a phospholipid of a liposome after completion thereof while controlling the density thereof.

When a sugar chain is bonded by use of a linker protein, the linker protein includes animal serum albumin including human serum albumin (HSA) and bovine serum albumin (BSA). Particularly when human serum albumin is used, it has been experimentally confirmed to show a large uptake by mice tissues.

When a targeting liposome according to the present invention is used as a pharmaceutical drug as is described later, it must contain a compound having a medicinal effect. The compound having a medicinal effect is encapsulated in the liposome or bonded to the surface of the liposome.

A sugar chain may be bonded to a liposome via a linker protein by the method mentioned below.

First, a protein is bonded to the surface of a liposome. The liposome is treated with an oxidizing agent such as NaIO₄, Pb(O₂CCH₃)₄, or NaBiO₃ to oxidize a ganglioside present on the membrane surface of the liposome. Then, the linker protein is bonded to the ganglioside on the liposome surface by a reductive amination using a reagent such as NaBH₃CN or NaBH₄. It is preferable that the linker protein is also hydrophilized by bonding a compound having a hydroxyl group to the linker protein. More specifically, a compound such as tris(hydroxymethyl)aminomethane for use in hydrophilization mentioned above may be bonded to a linker protein on a liposome by use of a divalent reagent such as bissulfosuccinimidylsuberate, disuccinimidylglutarate, dithiobissuccinimidylpropionate, disuccinimidylsuberate, 3,3'-dithiobissulfosuccinimidylpropionate, ethylene glycol bissuccinimidylsuccinate, or ethylene glycol bissulfosuccinimidylsuccinate.

To describe this more specifically, one of the ends of a divalent crosslinking reagent is bonded to all amino groups of a linker protein. Then, the reduced terminals of sugar chains are glycosylaminated to prepare a sugar chain glycosylamine compound. The amino groups of the sugar chains are bonded to the other unreacted terminals of part of the divalent crosslinking reagent bonded onto the liposome.

Subsequently, using the unreacted terminals (constituting major part) of the divalent reagent remaining intact on the surface of the protein on the sugar-chain bonded liposome membrane, hydrophilization treatment is performed. More specifically, the unreacted terminals of the divalent reagent bonded to the protein on the liposome are reactively bonded with a hydrophilic compound such as tris(hydroxymethyl)aminomethane, thereby hydrophilizing the entire surface of the liposome.

The hydrophilization of the surface of a liposome and a linker protein improves delivery of the liposome to various types of tissues and blood retention and delivery of the liposome to various types of tissues for the reasons below. It is conceivable that the hydrophilization of the surfaces of the liposome and the linker protein causes tissues to recognize the portion except for the sugar chain as an *in-vivo* moisture content, with the result that other tissues except for a target tissue fail to recognize the liposome and only the sugar chain is recognized by a lectin (sugar chain recognition protein) of the target tissue.

Subsequently, the sugar chain is bonded to a linker protein on the liposome. This is performed by glycosylaminating the reduced terminal of a saccharide constituting the sugar chain by an ammonium salt such as NH₄HCO₃ or NH₂COONH₄, and then, bonding the linker protein bonded onto liposome membrane to the saccharide glycosylaminated in the above by use of a divalent reagent such as bissulfosuccinimidylsuberate, disuccinimidylglutarate, dithiobissuccinimidylpropionate, disuccinimidylsuberate, 3,3'-dithiobissulfosuccinimidylpropionate, ethylene glycol bissuccinimidylsuccinate, or ethylene glycol bissulfosuccinimidylsuccinate. As a result, the liposomes shown in Figures 1 to 22 can be obtained. These sugar chains are commercially available.

The particle size of the liposome of the present invention is 30 to 500 nm, preferably 50 to 300 nm, more preferably 70 to 150 nm. The zeta potential of the liposome of the present invention is -50 to 10 mV, preferably -40 to 0 mV, more preferably -30 to -10 mV. While the process for producing the liposome comprised in a pharmaceutical composition of the present invention can yield 4 types of liposomes, that is, a liposome with a surface to which no substance is bonded, an unhydrophilized liposome modified by a sugar chain is bonded, a hydrophilized liposome having no sugar chain bound thereto, and a hydrophilized liposome modified by a sugar chain, the particle sizes and zeta potentials of these 4 types of liposomes are adjusted to fall in the above-described ranges, by use of an isotonic solution such as a physiological salt solution.

When a sugar chain is bonded, the binding density of the sugar chain falls within the range 1 to 60 chains per molecule of a linker protein, preferably 1 to 40, and further preferably 1 to 20. When a linker protein is used, the binding density falls within the range of 1 to 30000 per liposome particle, preferably 1 to 20000, and further preferably, 1 to 10000; 100 to 30000, preferably 100 to 20000, and further preferably 100 to 10000; or 500 to 30000, preferably 500 to 20000, and further preferably 500 to 10000. When no linker protein is used, the number of sugar chains that may be bonded is 1 to 500000 per liposome particle, preferably 1 to 300000, and further preferably, 1 to 100000 or more, in maximum.

### (2) Composition for Medical Treatment of Inflammatory Disease Comprising Targeting Liposome of the Present Invention

The present invention encompasses a pharmaceutical composition for the medical treatment or diagnosis of an inflammatory disease comprising the targeting liposome in which the sialyl Lewis X sugar chain or the sugar chain that can be reacted with E-selectin, P-selectin, or the like as in the sialyl Lewis X sugar chain is bound to its membrane in a manner that controls the type and density of the sugar chain. Moreover, the present invention also encompasses a pharmaceutical composition for the medical treatment or diagnosis of an inflammatory disease comprising a liposome that is hydrophilized with the hydrophilic compound and has no sugar chain bound thereto.

Examples of inflammatory diseases targeted by the pharmaceutical composition for the medical treatment or diagnosis of an inflammatory disease of the present invention include, but not limited to, general inflammatory diseases such as encephalitis, inflammatory eye disease, otitis, pharyngitis, pneumonia, gastritis, enteritis, hepatitis, pancreatitis, nephritis, cystitis, urethritis, endometritis, vaginitis, arthritis, and peripheral neuritis, and further include inflammatory diseases that secondarily cause inflammation, such as malignant tumor, infectious diseases, allergic diseases, autoimmune diseases (such as rheumatism, systemic lupus erythematosus, and sarcoidosis), ischemic diseases (such as myocardial infarction and cerebral infarction), metabolic diseases (such as diabetes and gout), injury, scald, chemical corrosion, and neurodegenerative diseases (such as Alzheimer's disease). The pharmaceutical composition for the medical treatment or diagnosis of an inflammatory disease is applicable without limitations to any target organ or tissue, and is capable of targeting all organs and tissues in animals. Examples of the inflammatory eye disease include, but not limited to, intraocular inflammation including uveitis, diabetic retinopathy, neovascular maculopathy, allergic conjunctivitis, orbital and intraocular tumors, optic neuritis, and scleritis including posterior scleritis.

The targeting liposome comprised in the pharmaceutical composition for the medical treatment or diagnosis of an inflammatory disease of the present invention comprises a compound having pharmaceutical effects for the medical treatment of an inflammatory disease. Examples of the compound include, but not limited to, adrenocortical hormones, antiinflammatory drugs, immunosuppressive drugs, anticancer drugs, antimicrobial drugs, antiviral drugs, angiogenesis inhibitors, cytokines, chemokines, anti-cytokine antibodies, anti-chemokine antibodies, anti-cytokine/chemokine receptor antibodies, nucleic acid preparations for therapy using genes such as siRNA and DNA, and neuroprotective factors.

Examples of the adrenocortical hormones include natural adrenocortical hormones (glucocorticoids) such as cortisol and cortisone as well as synthetic adrenocortical hormones such as prednisolone, prednisolone phosphate, dexamethasone, dexamethasone phosphate, betamethasone, fluocinolone acetonide, and triamcinolone. Examples of other antiinflammatory drugs include salicylic acids such as aspirin, indoleacetic acid derivatives such as indomethacin, phenylacetic acid derivatives such as diclofenac, and fenamacetic acid derivatives such as mefenamic acid, and further include selective COX-2 inhibitors such as etodolac, meloxicam, celecoxib, refecoxib, and MK-0966.

In the present invention, these drugs encompass their derivatives.

When the sugar-modified liposome of the present invention incorporating any of these drugs is administered, the drug is accumulated in an inflammatory site at a level higher than that of the drug administered alone, and can be accumulated in an inflammatory site at a level 2 or more times, preferably 5 or more times, more preferably 10 or more times, particularly preferably 50 or more times, higher than that of the drug administered alone.

While these compounds may be encapsulated in the liposome or bound to the surface of the liposome, it is preferable to encapsulate the compounds in the liposome. These compounds can be bound thereto through a method known in the art by utilizing functional groups of the compounds.

The encapsulation of the compounds into the liposome is carried out through a method described below. The drug or the like may be encapsulated into the liposome through a conventionally known method. For example, a solution containing the drug or the like as well as a lipid mixture comprising phosphatidylcholines, phosphatidylethanolamines, phosphatidic acids or long-chain alkyl phosphates, gangliosides, glycolipids, or phosphatidylglycerols, and cholesterols is used to form liposome, thereby encapsulating the drug or the like into the liposome.

The pharmaceutical composition of the present invention may contain, for example, a pharmacologically acceptable carrier, diluent, or excipient, in addition to the targeting liposome in which the sialyl Lewis X sugar chain or the sugar chain that can be reacted with E-selectin, P-selectin, or the like as in the sialyl Lewis X sugar chain is bound to its surface in a manner that controls the type and density of the sugar chain, or the liposome that is hydrophilized with the hydrophilic compound and has no sugar chain bound thereto. A pharmaceutical composition of the present invention may be administered in various forms. Examples of such administration forms include instillation by an eyedrop; peroral administration by a tablet, capsule, granule, powder, and syrup; and parenteral administration by injection, drip infusion, and suppository. These compositions are produced by known methods and contain carriers, diluents and excipients generally used in the field of medicinal preparation. As carriers and excipients for tablets, use may be made of gelation agents, lactose, and magnesium stearate. Injection agents are prepared by dissolving, suspending or emulsifying the sugar chain bonded liposomes of the present invention in an aseptic aqueous or oily solution that is generally used in an injection agent. As the aqueous solution for injection, use may be made of physiological saline and isotonic solutions containing glucose and other auxiliary agents. In this case, an appropriate dissolution auxiliary agent(s), such as an alcohol, polyalcohol e.g., propylene glycol, and nonionic surfactant may be used simultaneously. As the oily solution, use may be made of sesame oil and soybean oil. As the dissolution auxiliary agent, use may be simultaneously made of benzyl benzoate and benzyl alcohol.

The administration route of a pharmaceutical composition of the present invention is not limited and includes instillation, peroral administration, intravenous injection and intramuscular injection. The administration amount can be appropriately determined depending upon significance of the inflammatory disease. In the present invention, a pharmaceutically effective amount of composition is administered to a patient. The phrase "a pharmaceutically effective amount is administered" refers to administering a medicinal drug in an appropriate amount for treating a inflammatory disease. The administration number of times of a pharmaceutical composition of the present invention is appropriately selected depending upon the symptom of a patient.

When the pharmaceutical composition of the present invention is used for diagnosis, a labeling compound such as a fluorescent pigment, or radioactive compound is bonded to liposomes. When the liposomes tagged with a labeling compound are bonded to a lesion, the labeling compound is taken in lesion cells. Therefore, a disease can be detected and diagnosed based on the presence of the targeting compound as an index.

The present invention will be explained in more detail by way of Examples, which should not be construed as limiting the present invention.

### EXAMPLE 1

### Preparation of Liposomes

Liposomes were prepared through an improved type of cholate dialysis based on a previously reported method (Yamazaki, N., Kodama, M. and H.-J. Gabius. *Methods Enzymol.* 242:56-65 (1994)). More specifically, 46.9 mg of sodium cholate was added to 45.6 mg of lipid mixture consisting of dipalmitoylphosphatidylcholine, cholesterol, dicetylphosphate, ganglioside and dipalmitoylphosphatidylethanolamine at a mole ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain a clear micelle suspension. Then, this micelle suspension was subjected to ultrafiltration by using a PM 10 membrane (Amicon Co., USA) and a PBS buffer solution (pH 7.2) to prepare 10 ml of a uniform liposome (average size of 100 nm).

### EXAMPLE 2

### Hydrophilization of Lipid Membrane Surface of Liposomes

10 ml of the liposome solution prepared in Example 1 was subjected to ultrafiltration by using an XM 300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the reaction between the BS3 and the dipalmitoylphosphatidyletanolamine of the lipid on the liposome membrane. This liposome solution was then subjected to ultrafiltration by using an XM 300 membrane and a CBS buffer solution (pH 8.5). Then, 40 mg of tris (hydroxymethyl) aminomethane dissolved in 1 ml of CMS buffer solution (pH 8.5) was added to 10 ml of the liposome solution. The obtained solution was stirred at 25°C for 2 hours, and stirred at 7°C for one night to complete the reaction between the BS3 bonded to the lipid on the liposome membrane and the tris (hydroxymethyl) aminomethane. In this manner, the hydroxyl groups of the tris (hydroxymethyl) aminomethane were coordinated on the dipalmitoylphosphatidyletanolamine of the lipid on the liposome membrane to achieve the hydrophilization of the lipid membrane surface of the liposome.

### EXAMPLE 3

### Bonding of Human Serum Albumin (HSA) to Membrane Surface ofLiposomes

Human serum albumin (HSA) was bonded to the membrane surface of the liposome through a coupling reaction method based on a previously reported method (Yamazaki, N., Kodama, M. and H. -J. Gabius. *Methods Enzymol.* 242:56-65 (1994)). More specifically, the reaction was carried out through a two-stage reaction method. That is, 43 mg of sodium metaperiodate dissolved in 1 ml of TAPS buffer solution (pH 8.4) was added to 10 ml of the liposome obtained in Example 2, and the obtained solution was stirred at room temperature for 2 hours to periodate-oxidize the ganglioside on the membrane surface of the liposome. Then, the solution was subjected to ultrafiltration by using an XM 300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 µl of 2M NaBH₃CN was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM 300 membrane and a CBS buffer solution (pH 8.5).

### EXAMPLE 4

Bonding of Lewis X Trisaccharide, Sialyl Lewis X Tetrasaccharide, 3'-Sialyllactosamine Trisaccharide, 6'-Sialyllactosamine Trisaccharide, α-1,2-Mannobiose Disaccharide, α-1,3-Mannobiose Disaccharide, α-1,4-Mannobiose Disaccharide, α-1,6-Mannobiose Disaccharide, α-1,3/α-1,6-Mannotriose Trisaccharide, Oligomannose-3 Pentasaccharide, Oligomannose-4b Hexasaccharide, Oligomannose-5 Heptasaccharide, Oligomannose-6 Octasaccharide, Oligomannose-7 Nonasaccharide, Oligomannose-8 Decasaccharide, or Oligomannose-9 Undecasaccharide, to Human Serum Albumin (HSA) Bound on Liposome Membrane Surfaces

50 µg of Lewis X Trisaccharide (Calbiochem Co.., USA) was added to 0.5 ml of water solution having 0.25 g of NH₄HCO₃ dissolved therein, and the obtained solution was stirred at 37°C for 3 days. Then, the solution was filtered by using a filter of 0.45 µm to complete an amination reaction at the reduction terminal of the sugar chain and obtain 50 µg of glycosylamine compound of the Lewis X Trisaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 µg of the glycosylamine compound of the Lewis X Trisaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the Lewis X Trisaccharide to the DTSSP on the human serum albumin bonded on the liposome membrane surface. In this manner, 2 ml of a liposome, in which Lewis X Trisaccharide is bonded to the liposome through human serum albumin (total lipid mass: 2 mg, total protein mass: 200 µg, average particle size: 100 nm), were obtained. Other sugar-chain bonded liposomes were produced in the same method by changing the sugar chain used. These sugar-chain bonded liposomes are shown in Figures 2 to 16.

### EXAMPLE 5

Bonding of Lactose Disaccharide, 2'-Fucosyllactose Trisaccharide, Difucosyllactose Tetrasaccharide, 3-Fucosyllactose Trisaccharide, 3'-Sialyllactose Trisaccharide, 6'-Sialyllactose Trisaccharide, 3'-Sialyllactosamine Trisaccharide, or 6'-Sialyllactosamine Trisaccharide to Human Serum Albumin (HSA) Bound on Liposome Membrane Surfaces (3 Types of Liposomes, Differing in Amount of Sugar Chain Bound Thereto)
(1) 50 µg, (2) 200 µg, or (3) 1 mg of lactose disaccharide (Wako Pure Chemical Co., Japan) was added to 0.5 ml of water solution having 0.25 g of NH₄HCO₃ dissolved therein, and the obtained solution was stirred at 37°C for 3 days. Then, the solution was filtered by using a filter of 0.45 µm to complete an amination reaction at the reduction terminal of the sugar chain and obtain 50 µg of glycosylamine compound of the lactose disaccharide. Then, 1 mg of 3,3'-dithiobis(sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bound to the HSA on the liposome. Then, 50 µg of the glycosylamine compound of the lactose disaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the lactose disaccharide to the DTSSP on the human serum albumin bound on the liposome membrane surface. In this manner, 3 types of liposomes (2 ml each), differing in the amount of sugar chain bound thereto, in which lactose disaccharide is bound to the liposome through human serum albumin (FIG. 17) (total lipid mass: 2 mg, total protein mass: 200 µg, average particle size: 100 nm), were obtained. Other sugar-modified liposomes were prepared in the same way with the exception that a sugar chain used was changed. The structures of other sugar-modified liposomes are shown in Figs. 3, 4, and 18 to 22.

### EXAMPLE 6

### Bonding of Tris (Hydroxymethyl) Aminomethane to Human Serum Albumin (HSA) Bonded on Liposome Membrane Surfaces

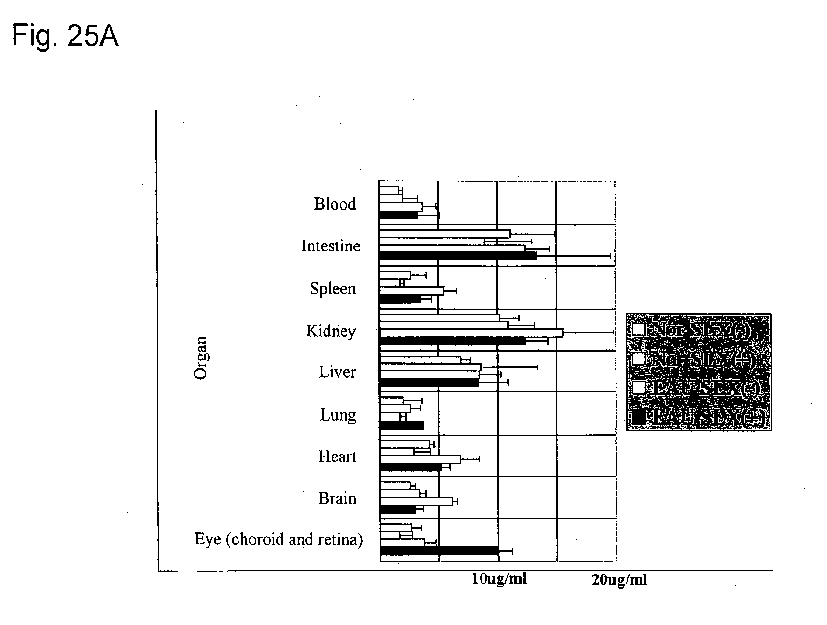

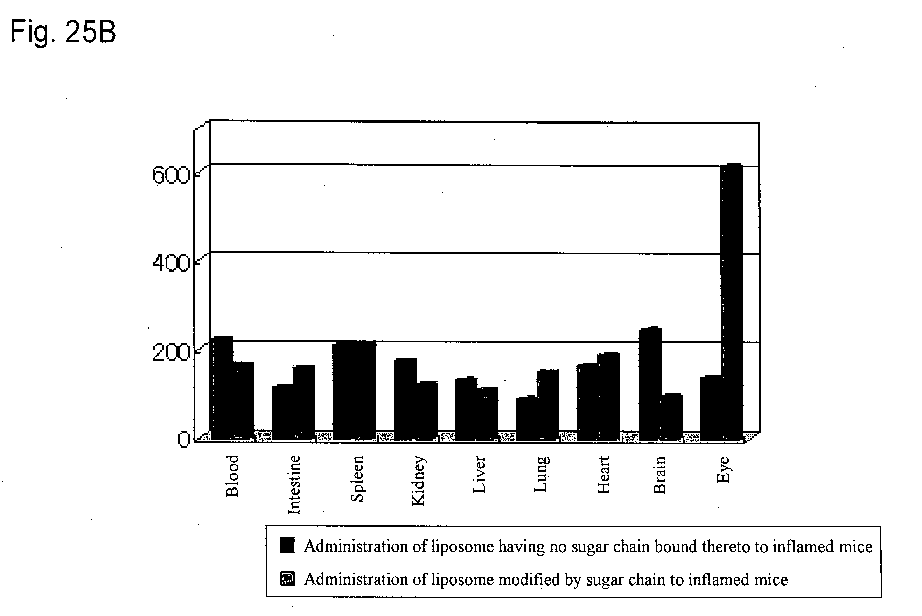

For preparing a liposome as a comparative sample, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was stirred at 25°C for 2 hours, and subsequently stirred at 7°C for one night. The solution was then subjected to ultrafiltration by using an XM 300 membrane and a CBS buffer solution (pH 8.5) to obtain I ml of liposome in which the DTSSP was bonded to the HSA on the liposome. Then, 13 mg oftris (hydroxymethyl) aminomethane (Wako Co., Japan) was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the tris (hydroxymethyl) aminomethane to the DTSSP on the human serum albumin bonded on the liposome membrane surface. In this process, an excess amount of tris (hydroxymethyl) aminomethane, that is 13 mg, already exists. Thus, the hydrophilization of the human serum albumin (HSA) bonded on the liposome membrane surface was simultaneously completed. In this manner, 2 ml of the liposome as the comparative sample (TRIS) in which the tris (hydroxymethyl) aminomethane is bonded to human serum albumin (FIG. 25) (total lipid mass: 2 mg, total protein mass: 200 µg, average particle size: 100 nm) was obtained.

### EXAMPLE 7

### Hydrophilization of Human Serum Albumin Bonded on Liposome Membrane Surfaces

For the liposomes prepared in Examples 4 or 5, the respective HSA protein surfaces were separately hydrophilized through the following process. 13 mg of tris (hydroxymethyl) aminomethane was added to each of the 12 types of sugar-modified liposomes (2 ml each). The respective obtained solutions were stirred at 25°C for 2 hours, and subsequently stirred at 7°C for one night. The solutions were then subjected to ultrafiltration by using an XM 300 membrane and a PBS buffer solution (pH 7.2) to remove unreacted materials. In this manner, 2 ml of final product for each of the 12 types of hydrophilized sugar-modified liposome complexes (total lipid mass: 2 mg, total protein mass: 200 µg, average particle size: 100 nm) were obtained.

### EXAMPLE 8

### Measurement of Lectin-Binding Activity Inhibiting Effect in Each Type of Sugar-Modified Liposome Complex

The in vitro lectin-binding activity of each of the 12 types of hydrophilized sugar-modified liposomes prepared in Examples 5 and 6 were measured through an inhibition test using a lectin-immobilized microplate by methods known in the art (see, e.g., Yamazaki, N., et al., *Drug Delivery System,* 14:498-505 (1999)). More specifically, a lectin (E-selectin; R&D Systems Co., USA) was immobilized on a 96 well-microplate. Then, 0.1 µg of biotinylated fetuin as a comparative ligand, and various types of sugar-modified liposome complexes having different densities (each including 0.01 µg, 0.04 µg, 0.11 µg, 0.33 µg or 1 µg of protein), were placed on the lectin-immobilized plate, and incubated at 4°C for 2 hours. After washing with PBS (pH 7.2) three times, horseradish peroxidase (HRPO)-conjugated streptavidin was added to each of the wells. The respective test solutions were incubated at 4°C for 1 hour, and then washed with PBS (pH 7.2) three times. Then, peroxidase substrates were added to the test solutions, and incubated at room temperature. Then, the absorbance at 405 nm of each of the test solutions was determined by a microplate reader (Molecular Devices Corp., USA). For the biotinylation of the fucosylated fetuin, each of the test solutions was subject to a sulfo-NHS-biotin reagent (Pierce Chemical Co., USA) treatment and refined by using a Centricon-30 (Amicon Co., USA). HRPO-conjugated streptavidin was prepared by oxidizing HRPO and bonding streptavidin to the oxidized HRPO through a reductive amination method using NaBH₃CN. This measurement results are shown in Tables 1 to 4.

Symbols in Tables denote that liposome is modified with sugar-chains below.
LX: Lewis X trisaccharide
SLX: sialyl Lewis X tetrasaccharide
3SLN: 3'-sialyllactosamine trisaccharide
6SLN: 6'-sialyllactosamine trisaccharide
A2: α-1,2-mannobiose disaccharide
A3: α-1,3-mannobiose disaccharide
A4: α-1,4-mannobiose disaccharide
A6: α-1,6-mannobiose disaccharide
A36: α-1,3/a-1,6-mannotriose trisaccharide
Man3: oligomannose-3 pentasaccharide
Man4: oligomannose-4b hexasaccharide
Man5: oligomannose-5 heptasaccharide
Man6: oligomannose-6 octasaccharide
Man7: oligomannose-7 nonasaccharide
Man8: oligomannose-8 decasaccharide
Man9: oligomannose-9 undecasaccharide
LAC: lactose disaccharide
2FL: 2'-fucosyllactose trisaccharide
DFL: difucosyllactose tetrasaccharide
3FL: 3-fucosyllactose trisaccharide
3 SL: 3'-sialyllactose trisaccharide
6SL: 6'-sialyllactose trisaccharide
3 SLN: 3'-sialyllactosamine trisaccharide
6SLN: 6'-sialyllactosamine trisaccharide

As shown in the tables, each of the liposomes has lectin-binding activity. This indicates that the sugar chains are bound to the membrane surfaces of the liposomes

**Table 1**

| Liposome complex | Inhibiting effect (absorbance) at each density of liposome complexes (µg protein) | | | | |
|---|---|---|---|---|---|
| | 0.01 µg | 0.04 µg | 0.11 µg | 0.33 µg | 1 µg |
| LX | 0.199 | 0.195 | 0.195 | 0.195 | 0.129 |
| SLX | 0.105 | 0.100 | 0.100 | 0.084 | 0.073 |
| 3SLN | 0.175 | 0.158 | 0.144 | 0.131 | 0.095 |
| 6SLN | 0.256 | 0.245 | 0.233 | 0.200 | 0.151 |

**Table 2**

| Liposome complex | Inhibiting effect (absorbance) at each density of liposome complexes (µg protein) | | | | |
|---|---|---|---|---|---|
| | 0.006 µg | 0.02 µg | 0.06 µg | 0.17 µg | 0.5 µg |
| A2 | 0.192 | 0.196 | 0.192 | 0.169 | 0.155 |
| A3 | 0.178 | 0.178 | 0.178 | 0.170 | 0.142 |
| A4 | 0.192 | 0.196 | 0.192 | 0.175 | 0.153 |
| A6 | 0.182 | 0.196 | 0.182 | 0.169 | 0.151 |
| A36 | 0.205 | 0.215 | 0.205 | 0.192 | 0.150 |
| Man3 | 0.201 | 0.211 | 0.201 | 0.177 | 0.144 |
| Man4 | 0.171 | 0.203 | 0.171 | 0.157 | 0.148 |
| Man5 | 0.215 | 0.221 | 0.215 | 0.196 | 0.164 |
| Man6 | 0.210 | 0.222 | 0.210 | 0.207 | 0.125 |
| Man7 | 0.213 | 0.214 | 0.213 | 0.183 | 0.137 |
| Man8 | 0.211 | 0.216 | 0.211 | 0.188 | 0.132 |
| Man9 | 0.208 | 0.211 | 0.208 | 0.186 | 0.135 |

**Table 3**

| Liposome complex | Inhibiting effect (absorbance) at each density of liposome complexes (µg protein) | | | | |
|---|---|---|---|---|---|
| | 0.01 µg | 0.04µg | 0.11 µg | 0.33 µg | 1 µg |
| LAC-1 | 0.115 | 0.114 | 0.112 | 0.112 | 0.105 |
| LAC-2 | 0.112 | 0.109 | 0.104 | 0.104 | 0.097 |
| LAC-3 | 0.119 | 0.118 | 0.112 | 0.109 | 0.108 |
| 2FL-1 | 0.121 | 0.115 | 0.106 | 0.097 | 0.067 |
| 2FL-2 | 0.131 | 0.119 | 0.116 | 0.111 | 0.079 |
| 2FL-3 | 0.149 | 0.133 | 0.122 | 0.104 | 0.073 |
| DFL-1 | 0.167 | 0.158 | 0.146 | 0.131 | 0.108 |
| DFL-2 | 0.136 | 0.134 | 0.133 | 0.120 | 0.106 |
| DFL-3 | 0.163 | 0.150 | 0.134 | 0.118 | 0.097 |
| 3FL-1 | 0.138 | 0.131 | 0.121 | 0.113 | 0.085 |
| 3FL-2 | 0.148 | 0.134 | 0.128 | 0.123 | 0.092 |
| 3FL-3 | 0.149 | 0.134 | 0.129 | 0.128 | 0.110 |

**Table 4**

| Liposome complex | Inhibiting effect (absorbance) at each density of liposome complexes (µg protein) | | | | |
|---|---|---|---|---|---|
| | 0.01 µg | 0.04 µg | 0.11 µg | 0.33 µg | 1 µg |
| 3SL-1 | 0.154 | 0.147 | 0.135 | 0.120 | 0.097 |
| 3 SL-2 | 0.149 | 0.142 | 0.124 | 0.118 | 0.098 |
| 3SL-3 | 0.214 | 0.214 | 0.210 | 0.183 | 0.167 |
| 6SL-1 | 0.177 | 0.171 | 0.167 | 0.160 | 0.114 |
| 6SL-2 | 0.196 | 0.184 | 0.169 | 0.160 | 0.159 |
| 6SL-3 | 0.214 | 0.207 | 0.196 | 0.192 | 0.183 |
| 3 SLN-1 | 0.219 | 0.198 | 0.180 | 0.164 | 0.119 |
| 3 SLN-2 | 0.155 | 0.155 | 0.151 1 | 0.119 | 0.096 |
| 3SLN-3 | 0.216 | 0.198 | 0.187 | 0.146 | 0.132 |
| 6SLN-1 | 0.257 | 0.246 | 0.233 | 0.200 | 0.151 |
| 6SLN-2 | 0.250 | 0.250 | 0.230 | 0.199 | 0.158 |
| 6SLN-3 | 0.248 | 0.231 | 0.227 | 0.201 | 0.144 |

### EXAMPLE 9

### Preparation of Model Mice of Uveitis

The following materials were used for preparing model mice of experimental uveitis (experimental autoimmune uveoretinitis; hereinafter, referred to as EAU).
Experimental animals: C57BL/6 mice (female, 8 weeks of age)
Inoculated antigen: Human retina-specific protein IRBP (interphotoreceptor retinoid binding protein)
Synthetic Peptide: Sequence GPTHLFQPSLVLDMAKVLLD (1-20)
Adjuvant: Complete Freund's adjuvant (CFA) containing 6 mg/ml killed *Mycobacterium tuberculosis* strain H37Ra
Promoting adjuvant: Purified *Bordetella pertussis* toxin (PTX)

A water solution of the peptide and the adjuvant were mixed at a volume ratio of 1:1 1 to prepare an emulsion. This emulsion was subcutaneously inoculated into the dorsal regions of the feet and inguinal regions of female C57BL/6 mice (8 weeks of age) in the total amount of 200 µg/mouse (50 µg each for the regions). The purified *Bordetella pertussis* toxin was intraabdominally administered as an additional adjuvant to the mice in an amount of 100 ng/mouse. The degree of EAU onset was evaluated by observing the fundus of the eye, with the pupils dilated by use of 0.5% tropicamide and 0.5% phenylephrine hydrochloride.

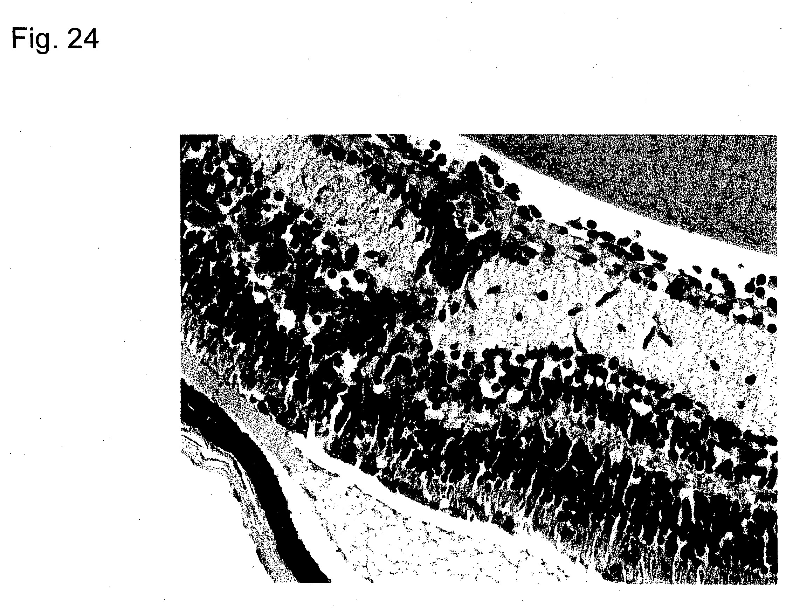

The result is shown in Fig. 24. The mice developed EAU that was at its peak on the 16th day from the administration.

It can be observed that inflammatory cells are infiltrated into the choroid, retina, and vitreous chamber on the 16th day from the administration of the peptide.

EAU serving as a human intraocular inflammation model was confirmed to be constructed by this method.

### EXAMPLE 10

### In Vivo Study ofLiposomes

Liposomes administered in this study were the sialyl Lewis X saccharide-modified liposome (hereinafter, referred to as sugar chain+ liposome) selected from the sugar-modified liposomes prepared in the above-described Examples and the liposome having no sugar chain bound thereto (hereinafter, sugar chain- liposome).

The sugar chain+ liposome and the sugar chain- liposome were respectively administered to each of the EAU mice and normal mice, for the purpose of evaluating the accumulation of the liposomes to each organ of the mice.

The EAU mice confirmed to develop EAU on the 16th day from the administration of the peptide as well as normal mice were prepared. A liposome solution prepared in advance to 50 µg/ml was injected into the tail veins of the mice. After this intravenous injection, blood was removed from the mice and returned thereto by using a physiological salt solution with heparin, and all organs were extracted. Each of the organs was prepared as tissue homogenates by using 1% Triton X solution and an HG30 homogenizer (Hitachi-Koki), from which the liposome was then extracted with 100% methanol and chloroform. The amount of the liposome was determined by measuring the fluorescence intensity (excitation at 490 nm and emission at 520 nm) of FITC bound to the liposome with a fluorescence microplate reader Biolumin 960 (Molecular Dynamics).

After the administration of the liposome, a temporal shift in the accumulation of the liposome in the eye was examined. As a result, the accumulation of the liposome was shown to be at its peak on 30 minutes from the administration. Thus, the study on accumulation in each organ was performed by setting time to 30 minutes from the administration of the liposome. The result is shown in Figs. 25A and 25B. Fig. 25A shows the accumulation of the liposome in each organ in terms of the amount of the liposome accumulated, while Fig. 25B shows it in terms of % relative value compared to in the normal mice. For the normal mice, no difference was observed between the accumulation of the sugar chain+ liposome and the accumulation of the sugar chain- liposome in each organ. On the other hand, the accumulation of the sugar chain+ liposome in the eyes of the EAU mice was observed to be 6 times its accumulation in the eyes of the normal mice. However, the accumulation of the sugar chain- liposome in the eyes of the EAU mice stayed at 1.5 times its accumulation in the eyes of the normal mice. No evident difference was observed between the accumulation of the sugar chain+ liposome and the accumulation of the sugar chain- liposome in the organs other than the eye of the EAU mice. Their values of accumulation were almost equal to those for the normal mice.

The uptake of the liposome was observed only in the inflamed eyes. The uptake of the sugar chain- liposome into the eyes of the EAU mice observed at a level 1.5 times higher than that into the eyes of the normal mice may be due to the expanded gaps between vascular endothelial cells (passive transport). On the other hand, the accumulation of the sugar chain+ liposome into the eyes of the EAU mice observed at a level 6 times higher than that into the eyes of the normal mice (i.e., the accumulation about 4 times the accumulation of the sugar chain- liposome) may be because its sugar chain targets E-selectin and P-selectin expressed in the inflammatory sites (active transport).

### EXAMPLE 11

### Accumulation of Liposomes to Inflammatory Sites

A test was performed for the purpose of evaluating which site of the inflammatory organ (eye) had the accumulation of the liposome.

The liposome was administered to the tail veins of the EAU mice on the 16th day after peptide immunization. After 30 minutes, the eyeballs were extracted from the mice and directly embedded in an OCT compound, which was then frozen. Frozen sections of 6 to 8 um were prepared using a cryotome and observed with Axio Vision (Carl Zeiss).

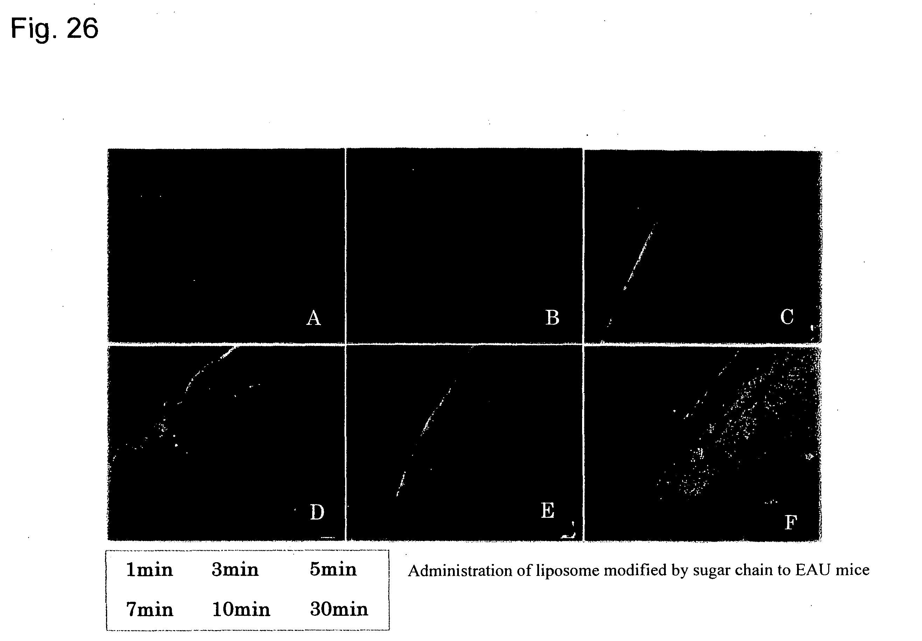

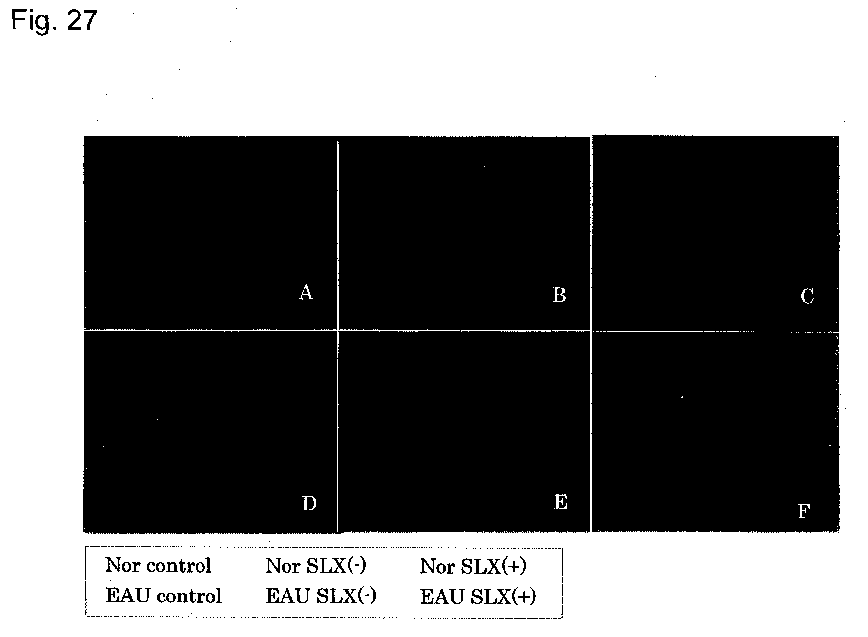

The result is shown in Fig. 26. When the sugar chain+ liposome was administered to the EAU mice, its accumulation was observed in the sclera after 5 minutes from the administration, and a thin streak of the accumulated liposome was observed in a site corresponding to the choriocapillary lamina of the choroid after 7 minutes from the administration. After 10 minutes from the administration, it can be observed that the line of the accumulated liposome is expanded. After 30 minutes from the administration, it can be observed that the line of the accumulated liposome is rendered thinner, and fluorescent substances are diffused to its neighboring tissues (Fig. 26). On the other hand, the line of the accumulated sugar chain- liposome is not observed, and the evident formation and diffusion of fluorescent substances can not be observed after 30 minutes from the administration (Fig. 26-F). As for the normal mice, neither of the liposomes was observed to form fluorescent substances (Fig. 27).

It was shown that the liposome behaves in the inflammatory site as follows: the sugar chain+ liposome starts to be accumulated in the choriocapillary lamina enriched most highly with blood vessels and is diffused to its neighboring tissues as time passes. This is possibly because the sugar chain+ liposome is accumulated as a result of targeting E-selectin and P-selectin expressed in the blood vessels of the inflammatory site, and then diffused to its neighboring tissues through the gap between vascular endothelial cells, which has been expanded by the inflammation. In contrast, a conceivable reason for no observation of evident fluorescent dyes for the sugar chain- liposome is that the accumulation of the liposome to the inflammatory site is passive transport. Therefore, unlike the sugar chain+ liposome, a site serving as a target for the sugar chain- liposome is absent, and this liposome is diffused in a piecemeal fashion, to the tissues.

### EXAMPLE 12

### Expression of E-selectin and P-selectin

A test was performed for the purpose of confirming whether E-selectin and P-selectin were certainly expressed in the eyes of the EAU mice and whether the site having their expression agreed with the line where the sugar chain+ liposome was first accumulated.

The EAU mice and normal mice were prepared. After the extraction of the eyeballs, the extracted eyeballs were immobilized on 4% glutaraldehyde and dehydrated with 30% sucrose/PBS. The resulting eyeballs were embedded in an OCT compound, which was then frozen.
(1) Detection through fluorescent antibody method: frozen sections of 14 to 16 µm were used and reacted, after blocking, with an anti-mouse E-selectin primary antibody (polyclonal antibody; gout). Signals were detected using an FITC-conjugated anti-gout IgG secondary antibody.
(2) Detection though enzyme antibody method: a reaction with an anti-mouse E-selectin and P-selectin primary antibody (polyclonal antibody; gout) was performed in the same way as in the fluorescent antibody method. A secondary antibody reaction was performed using VECTASTAIN (registered trademark) ABC-AP kit and Vector (registered trademark) Red Alkaline Phosphatase Substrate kit I.

In both of the detection methods, a peptide used in antibody production was purchased from each of the companies from which the antibodies had been purchased, in order to investigate the specificity of immunostaining, and this peptide was used as a blocking peptide to confirm whether staining would disappear or not.

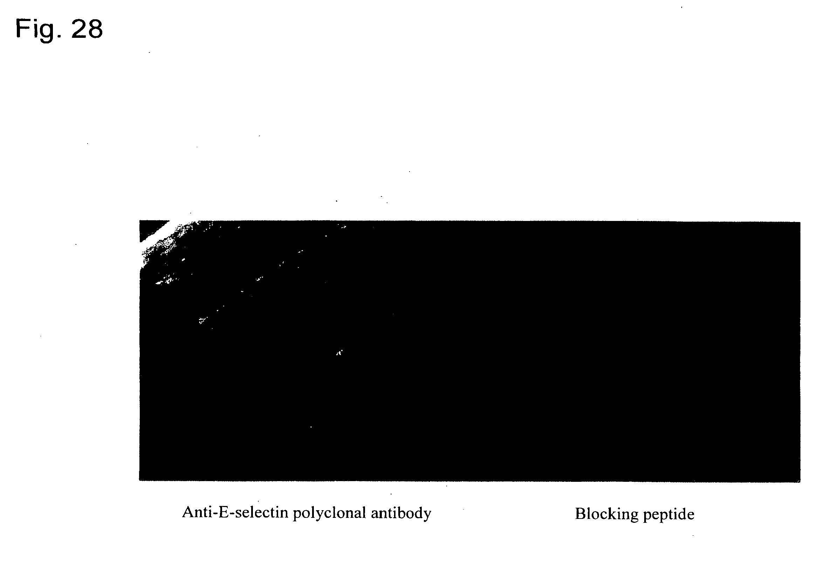

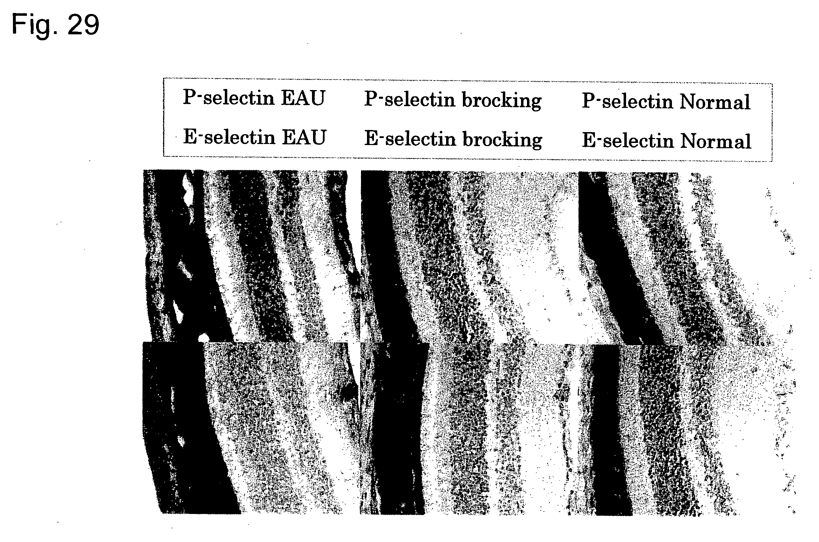

The results are shown in Figs. 28 and 29. It can be observed that both E-selectin and P-selectin are strongly expressed at a site that agrees with the line of the accumulated sugar chain+ liposome. Moreover, the staining disappeared by the respective neutralizing peptides for the antibodies used, indicating the specificity of this immunostaining. On the other hand, neither E-selectin nor P-selectin was observed to be expressed in the normal mice.

The agreement between the site where E-selectin and P-selectin are expressed and the site where the sugar chain+ liposome is accumulated, in the EAU mice supports the theory that targets for this liposome are E-selectin and P-selectin.

### EXAMPLE 13

### Inhibition of Accumulation of Sugar Chain+ Liposome by Administration of Antibodies against E-selectin and P-selectin

A test was performed for the purpose of verifying that molecules targeted by the sugar chain+ liposome were certainly E-selectin and P-selectin.

An anti-mouse E-selectin antibody (200 µg) and an anti-mouse P-selectin antibody (200 µg) were simultaneously administered to the tail veins of the EAU mice. After 4 hours, receptors were neutralized. Isotypic IgG (400 µg) was administered to the tail veins of the EAU mice, which were used as controls. After 4 hours of the administration, the sugar chain+ liposome was administered to the tail veins of the EAU mice. After 10 minutes, the eyeballs were extracted from the mice and prepared as frozen samples. Frozen sections of 6 to 8 µm were prepared and analyzed for the inhibition of ligand-receptor specific bonding by use of AxioVision.

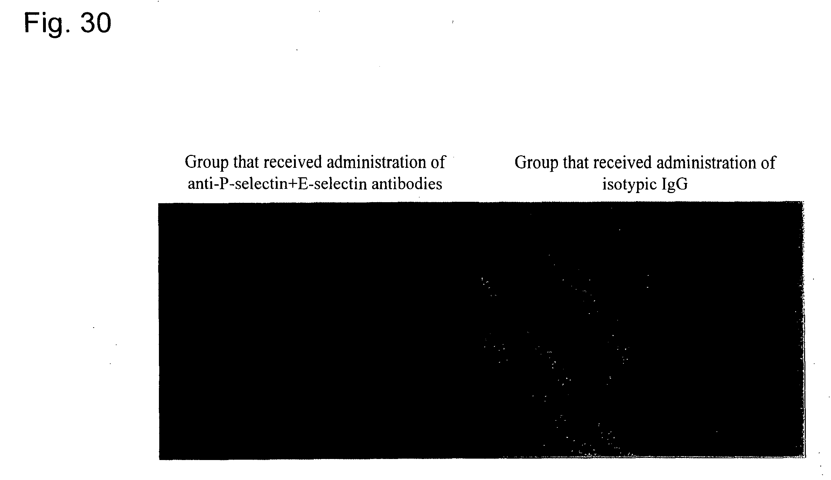

The result is shown in Fig. 30. The simultaneous administration of the antibodies against E-selectin and P-selectin to the EAU mice allowed the accumulation of the sugar chain+ liposome to be inhibited. The administration of the isotypic IgG did not allow its accumulation to be inhibited. When the respective antibodies against E-selectin and P-selectin were administered alone, the accumulation of the sugar chain+ liposome could not quite be inhibited.

From this result together with the result shown in Example 11, it can be concluded that the targets for the sugar chain+ liposome are E-selectin and P-selectin.

These test results demonstrated that the sialyl Lewis X tetrasaccharide-modified liposome is capable of specifically delivering drugs to lesions having the high expression levels of E-selectin and P-selectin.

### Example 14

### Preparation of Liposome Encapsulating Predonisolone Phosphate

### (1) Preparation of Liposome Encapsulating Predonisolone Phosphate, Quantification thereof and Storage Stability

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 10 ml of a TAPS buffered saline solution (pH 8.4), and was subjected to a supersonic treatment to obtain 10 ml of a clear micelle suspension. Then, predonisolone phosphate, completely dissolved in the TAPS buffer solution (pH 8.4) at 3 mg/ml, was added dropwise slowly to this micelle suspension while stirring. After being mixed homogeneously, this micelle suspension containing predonisolone phosphate was subjected to ultrafiltration by using a PM10 membrane (Amicon Co., USA) and the TAPS buffered saline solution(pH 8.4) to prepare 10 ml of a uniform suspension of the liposome encapsulating predonisolone phosphate. The liposome encapsulating predonisolone phosphate in the resulting physiological saline suspension (37°C) thus obtained was subjected to analysis for the particle diameter and zeta potential using a measuring device for zeta potential, particle diameter and molecular weight (Model Nano ZS, Malvern Instrumentss Ltd., UK) and the particle diameter was 50 to 350 nm and the zeta potential was -30 to -10 mV. The measurement of the amount of encapsulated drug by absorbance at 260nm revealed that about 280 µg/ml of predonisolone phosphate was encapsulated in the liposome. The liposome encapsulating predonisolone phosphate was stable without precipitation and aggregation after the storage in a refrigerator for one year.

### (2) Hydrophilization of Lipid Membrane Surface of Liposomes Encapsulating Predonisolone Phosphate

10 ml of the liposome solution containing predonisolone phosphate prepared in (1) above was subjected to ultrafiltration using an XM300 membrane (Amicon Co., USA) and a CBS buffer solution (pH 8.5) to adjust the pH of the solution to 8.5. Then, 10 ml of bis(sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the reaction between the BS3 and the dipalmitoylphosphatidyletanolamine of the lipid on the liposome membrane. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris(hydroxymethyl)aminomethane. In this manner, the hydroxyl groups of the tris(hydroxymethyl)aminomethane were coordinated on the lipid dipalmitoylphosphatidyletanolamine of the liposome membrane encapsulating predonisolone phosphate to achieve the hydrophilization thereof.

### (3) Bonding of Human Serum Albumin (HSA) to Membrane Surface of Liposomes Encapsulating Predonisolone Phosphate

A coupling reaction method based on a previously reported method (Yamazaki, N., Kodama, M. and Gabius, H. -J. (1994) Methods Enzymol. 242, 56-65) was used. More specifically, the reaction was carried out through a two-stage reaction method. That is, 43 mg of sodium metaperiodate dissolved in 1 ml of TAPS buffer solution (pH 8.4) was added to 10 ml of the liposome obtained in Example 2, and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 µl of 2M NaBH₃CN was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of the solution of the HSA-bonded liposome encapsulating predonisolone phosphate was obtained through an ultrafiltration using an XM300 membrane and a CBS buffer solution (pH 8.5).

### (4) Bonding of Sialyl Lewis X tetrasaccharide to Human Serum Albumin (HSA) Bonded on Liposome Membrane Surfaces and Hydrophilization of Linker Protein (HAS)

50 µg of sialyl Lewis X tetrasaccharide (Calbiochem Co., USA) was added to 0.5 ml of water solution having 0.25 g of NH₄HCO₃ dissolved therein, and the obtained solution was stirred at 37°C for 3 days. Then, the solution was filtered by using a filter of 0.45 µm to complete an amination at the reduced terminal of the sugar chain and obtain 50 µg of glycosylamine compound of sialyl Lewis X tetrasaccharide. Then, 1 mg of 3,3'-dithiobis (sulfosuccinimidyl propionate) (DTSSP; Pierce Co., USA) serving as a crosslinking reagent was added to 1 ml of a part of the solution of the liposome encapsulating predonisolone phosphate obtained in (3) above. The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of liposome in which the DTSSP was bonded to the HSA on the liposome. Then, 50 µg of the glycosylamine compound of sialyl Lewis X tetrasaccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (pH 7.2) to bond sialyl Lewis X tetrasaccharide to the DTSSP on the human serum albumin bonded on the liposome membrane surface. 13 mg of tris(hydroxymethyl)aminomethane was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. As the result, the liposome was obtained by hydrolizing the linker protein (HSA) in which tris(hydroxymethyl)aminomethane, human serum albumin and liposome were bonded. In this manner, 2 ml of the liposome encapsulating predonisolone phosphate with the hydrophilized linker protein (HSA), in which sialyl Lewis X tetrasaccharide, human serum albumin and liposome were bonded, (abbreviated as: DX-A6) (total lipid mass: 2 mg, total protein mass: 200 µg) was obtained. The suspension of the liposome encapsulating predonisolone phosphate in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

### (5) Hydrophilization of Linker Protein (HSA) by the Bonding of Tris(hydroxymethyl)aminomethane to Human Serum Albumin (HSA) Bonded on Membrane Surfaces of Liposome Encapsulating Predonisolone Phosphate

To prepare liposome encapsulating predonisolone phosphate (without sugar chain) as a comparative sample, 1 mg of 3, 3'-dithiobis (sulfosuccinimidyl) propionate (DTSSP; Pierce Co., USA) serving as a crosslinking reagent was added to 1 ml of a part of the solution of the liposome encapsulating predonisolone phosphate obtained in (3) above. The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome with the hydrophylized linker protein (HSA) in which DTSPP was bonded to the HSA on the liposome. 13 mg of tris(hydroxymethyl)aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome encapsulating predonisolone phosphate (without sugar chain) with the hydrophilized linker protein (HSA), in which tris(hydroxymethyl)aminomethane, human serum albumin and liposome were bonded, (abbreviated as DX-TRIS) (total lipid mass: 2 mg, total protein mass: 200 µg) was obtained. The suspension of the liposome encapsulating predonisolone phosphate in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

The liposomes prepared in this Example were used in studies described in EXAMPLES 15 and 16 below.

### EXAMPLE 15

### Effect of Accumulating Drug to Inflammatory Site

A test was performed for the purpose of examining the expression of E-selectin in each organ of the EAU mice.

Prednisolone phosphate (1 mg) was administered to the tail veins of the mice. After 1 hour, a prednisolone concentration per unit weight in each organ confirmed to have the expression of E-selectin was measured by HPLC. Subsequently, prednisolone phosphate encapsulated in the E-selectin-targeting liposome modified by sialyl Lewis X saccharide (hereinafter, referred to as sugar-modified liposome) developed this time was administered to the EAU mice in an amount equivalent to a total of 5 µg of prednisolone phosphate. After 1 hour, a prednisolone concentration per unit weight in each organ was measured by HPLC.

The following results are obtained.
1. The expression ofE-selectin was observed in the intestine of the EAU mice, in addition to the eye. Its expression was not observed in the other organs.
2. When 1 mg of prednisolone phosphate was administered alone, 100 ng and 500 ng of prednisolone were accumulated in the eye and intestine, respectively. On the other hand, when the sugar-modified liposome (incorporating a total of 5 µg of prednisolone phosphate therein) was administered, 25 ng and 2000 ng of prednisolone were accumulated in the eye and intestine, respectively.

The result obtained in this Example indicates that the use of the sugar-modified liposome incorporating 20 µg of prednisolone phosphate (for eye inflammation) or only 1.25 µg of prednisolone phosphate (for intestine inflammation) achieves an effect equal to the effect produced by the single administration of 1 mg of prednisolone phosphate, in the inflammatory site. Namely, when this sugar-modified liposome is used in a delivery system, 50-fold and 800-fold accumulating effects are obtained in the eye and intestine, respectively.

In this Example, while prednisolone phosphate was administered, measurement was performed relative to prednisolone. Because prednisolone phosphate taken into cells is converted to prednisolone by the removal of a phosphate group, the result obtained in this Example also indicates that the prednisolone phosphate encapsulated in the sugar-modified liposome is taken into cells and metabolized therein.

In summary, the tests using the EAU mice demonstrated that this sugar-modified liposome selectively targets E-selectin and P-selectin and exhibits up to 800-fold accumulating effect, which is a highly accurate targeting performance never before seen. Moreover, it could also be confirmed that a drug encapsulated in this sugar-modified liposome is metabolized in cells. As above, it can be concluded that a drug delivery system using the sugar-modified liposome of the present invention can decrease the amount of a drug administered to a few hundredth of a conventional amount, in order to obtain a therapeutic effect used in previous clinical medicine, and achieves the highest possible therapeutic effect and the fewest possible side effect.

### EXAMPLE 16

### Study on Medical Treatment of Rheumatoid Arthritis with Targeting Liposomes

### (1) Preparation of RA model mouse

Collagen type II induced arthritis (hereinafter, referred to as CIA) model was prepared as a mouse model of RA. Mice and reagents used are as follows:
Experimental animals: DBA/1J mice (male, 8 weeks of age)
Inoculated antigen: Bovine collagen type II
Adjuvants: Complete Freund's adjuvant (CFA) containing 2 mg/ml killed *Mycobacterium tuberculosis* strain H37Ra and incomplete Freund's adjuvant (IFA) free of killed *Mycobacterium tuberculosis*

A water solution of the collagen and the CFA were mixed at a volume ratio of 2:1 to prepare an emulsion containing 200 µg of the collagen per 100 µL thereof This emulsion was subcutaneously injected into the bottom of the tails of the mice (Day 0). After 21 days from the administration (Day 21), a water solution of the collagen and the IFA were mixed at a volume ratio of 2:1 to prepare an emulsion containing 200 µg of the collagen per 100 µL thereof, and this emulsion was subcutaneously injected again into the bottom of the tails of the mice.

After the injection, the limbs of the mice were observed on a three times/week basis, and inflammation was quantitatively determined by giving it a point according to the following criteria: for each limb, normal: 0 point, mild inflammation or redness: 1 point, severe redness, dilatation, or reduced use: 2 points, and deformed palm, plantar region, or joint region: 3 points (0 point lowest, 12 points highest, per mouse). Inflammatory activity (hereinafter, referred to as IA) was determined by applying the obtained point to the following formula: Inflammatory activity (%)=points of all limbs/12x100

The following results are obtained.

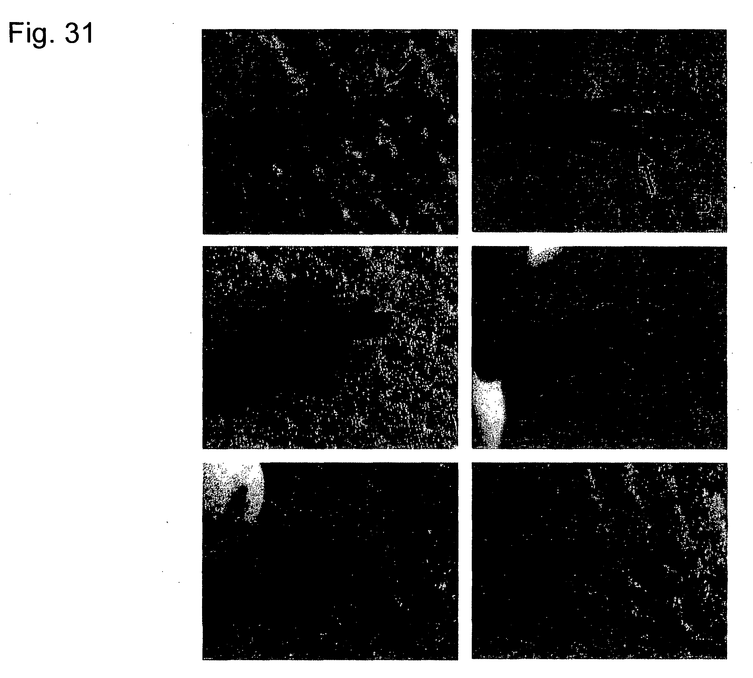

The number of mice that could be confirmed to have toe redness was increased in a few days from Day 21. The IA of the mice reached 16.7% on Day 28 and 50% on Day 39. A photograph showing the inflamed limbs of the mice is shown in Fig. 31. Observations of arthritis in the CIA mice are as follows: A: the dilatation of a joint in the toe (second toe, indicated by the arrow); B: the dilatation of a joint in the toe (fourth toe, indicated by the arrow); C: the redness of a joint in the toe (indicated by the arrow); D: normal posterior limb; E: the dilatation of a joint in the finger (indicated by the arrow); and F: normal forefoot.

As above, the CIA mice could be prepared as a mouse model of RA by this method.

### (2) Test on medical treatment of RA mice by intravenous administration of DDS liposomes

Mice with arthritis received medical treatment by injecting a therapeutic drug to the tail veins thereof. The intravenous injection was performed on a twice/week basis for a period from Day 28 to Day 46.

The mice observed to have inflammation on Day 28 were selected and divided into 4 groups so that each of the groups had the same average inflammation point as those of the other groups. The breakdown of these 4 groups was as follows: 1) Control: an untreated group, 2) free Pred: a group receiving the intravenous injection of a water solution having prednisolone phosphate dissolved in a physiological salt solution, in an amount equivalent to 100 µg/injection (200 µg/week) of prednisolone phosphate, 3) L-Pred: a group receiving the intravenous injection of prednisolone phosphate encapsulated in the liposome having no sugar chain bound thereto, in an amount equivalent to 10 µg/injection (20 µg/week) of prednisolone phosphate, and 4) L-Pred-SLX: a group receiving the intravenous injection of prednisolone phosphate encapsulated in the liposome in the sialyl Lewis X tetrasaccharide-modified liposome, in an amount equivalent to 10 µg/injection (20 µg/week) of prednisolone phosphate.

The number of mice contained in each of these 4 groups was 6 mice for Control, 7 mice for free Pred, 8 mice for L-Pred, and 8 mice for L-Pred-SLX.

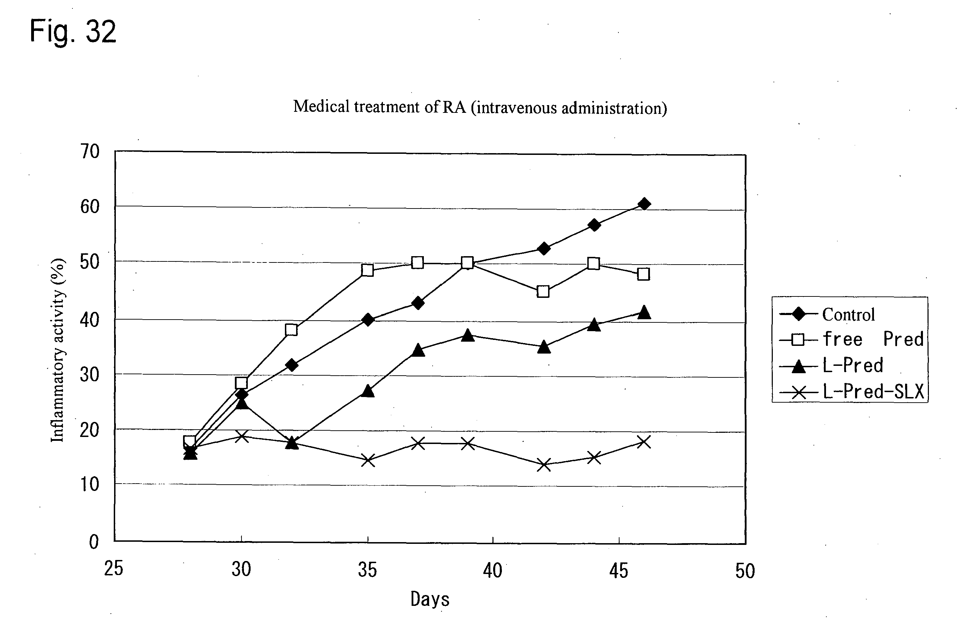

The following results were obtained. For the Control group, IA elevated over time and broke 60% at the time of Day 46; for the free Pred group, IA reached approximately 50% on Day 37 and then leveled off; for the L-Pred group, IA started to elevate on Day 32 and was approximately 40% on Day 46; and for the L-Pred-SLX group, no significant rise was observed in IA, which was 20% or low throughout the test days (Fig. 32).

The following became clear from this result.

The amount (100 µg/injection) of prednisolone phosphate intravenously injected to the free Pred group was insufficient to control inflammation in this group, and no evident difference was observed between the IA of the free Pred group and the IA of the Control group. Although the amount (10 µg/injection) of prednisolone phosphate intravenously injected to the L-Pred group was one-tenth of that to the free-Pred group, the IA of the L-Pred group was equal to or lower than that of the free Pred group. This seemed to indicate the possibility that the encapsulation of prednisolone phosphate into the liposome improved sustainability in blood of the drug, thereby improving the efficacy of the drug. Likewise, the amount (10 µg/injection) of prednisolone phosphate intravenously injected to the L-Pred-SLX group was one-tenth of that to the free-Pred group, and however, the L-Pred-SLX group had no rise in IA and had inflammation whose progression was significantly suppressed. This was considered to be an effect brought about by the difference between the L-Pred and L-Pred-SLX groups, that is, the presence or absence of the sialyl Lewis X tetrasaccharide. This result seemed to indicate the possibility that the sialyl Lewis X tetrasaccharide contributed to the effective accumulation of the liposome to the inflammatory site.

The result of the test demonstrated that the DDS liposome of the present invention has the ability to exceedingly effectively distribute the therapeutic drug to the inflammatory site. This suggests the possibility that the reduced side effect or enhanced efficacy of therapeutic drugs in the medical treatment of inflammatory diseases is achieved by focusing the therapeutic drugs on the lesions.

### (3) Test on medical treatment of RA mice by oral administration of DDS liposomes

Mice with arthritis received medical treatment by orally administering a therapeutic drug to the mice. The oral administration was performed on a three times/week basis for a period from Day 28 to Day 39.

The mice observed to have inflammation on Day 28 were selected and divided into 4 groups so that each of the groups had almost the same average inflammation point as those of the other groups. The breakdown of these 4 groups was as follows: 1) Control: an untreated group, 2) free Pred: a group receiving the oral administration of a water solution having prednisolone phosphate dissolved in a physiological salt solution, in an amount equivalent to 100 µg/injection (300 µg/week) of prednisolone phosphate, 3) L-Pred: a group receiving the oral administration of prednisolone phosphate encapsulated in the liposome having no sugar chain bound thereto, in an amount equivalent to 10 µg/injection (30 µg/week) of prednisolone phosphate, and 4) L-Pred-SLX: a group receiving the oral administration of prednisolone phosphate encapsulated in the sialyl Lewis X tetrasaccharide-modified liposome, in an amount equivalent to 10 µg/injection (30 µg/week) of prednisolone phosphate.

The number of mice contained in each of these 4 groups was 2 mice for Control, 3 mice for free Pred, 3 mice for L-Pred, and 4 mice for L-Pred-SLX.

The following results were obtained.

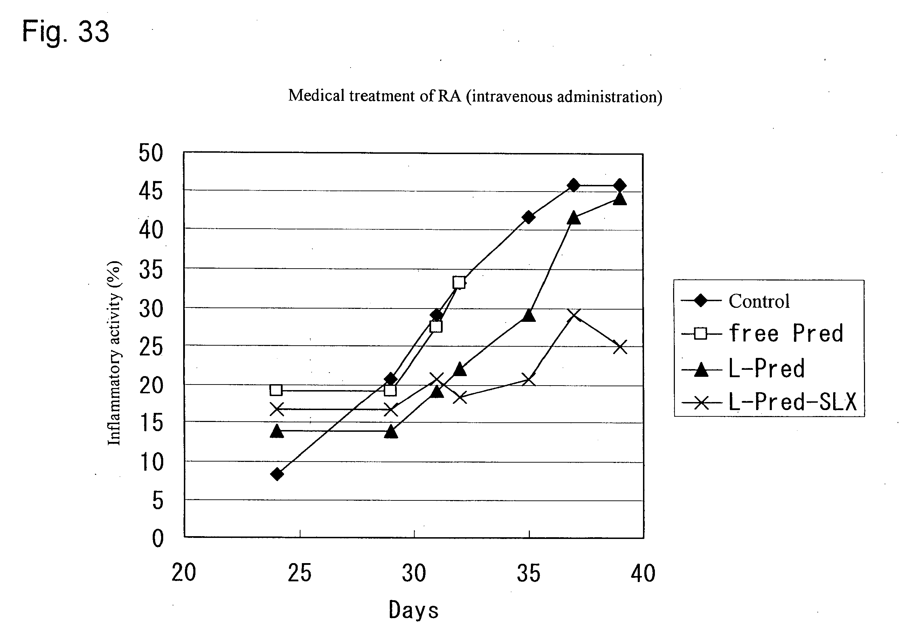

For the free Pred group, the test was discontinued on Day 32 because they died of gastric perforation caused by a feeding needle; no significant difference was observed among the IA of the control, free Pred (until Day 32), and L-Pred groups; and for the L-Pred-SLX group, while the progression of inflammation could not be suppressed, its IA was significantly low on Day 39, as compared with that of the L-Pred group (Fig. 33).

The following became clear from this result.

No significant difference was observed among 3 groups, the control, free-Pred, and L-Pred groups. This was conceivably because the amount (300 µg/week) of prednisolone phosphate orally administered to the free Pred group was insufficient to suppress the progression of inflammation in this group. Moreover, it was highly possible that the liposome orally administered to the L-Pred group was not absorbed at the intestine; or otherwise, considering that the amount of prednisolone phosphate orally administered was 30 µg/week, this amount was insufficient even if the liposome was absorbed at the intestine. In contrast, the IA of the L-Pred-SLX group was reduced. This seemed to suggest the possibility that the liposome orally administered to this group was absorbed at the intestine and, considering that the amount of prednisolone phosphate orally administered was 30 µg/week, the sugar-modified liposome incorporating the prednisolone phosphate therein was delivered intact to the inflammatory site.

This result indicates that the DDS liposome of the present invention effectively functions not only by intravenous administration but also by oral administration, and further suggests that a preparation that can be administered orally may be produced from a drug conventionally impossible to absorb via an oral route if the drug is encapsulated in the liposome.

### (4) Test for determining proper amount of prednisolone phosphate for medical treatment of RA mice

Mice with arthritis received medical treatment by injecting a therapeutic drug to the tail veins thereof. The intravenous injection was performed on a twice/week basis for a period from Day 28 to Day 46.

The mice observed to have inflammation on Day 28 were selected and divided into 3 groups so that each of the groups had almost the same average inflammation point as those of the other groups. The breakdown of these 3 groups was as follows: 1) Control: an untreated group, 2) 250 µg: a group receiving the intravenous injection of a water solution having prednisolone phosphate dissolved in a physiological salt solution, in an amount equivalent to 250 µg/injection (500 µg/week) of prednisolone phosphate, and 3) 500 µg: a group receiving the intravenous injection of a water solution having prednisolone phosphate dissolved in a physiological salt solution, in an amount equivalent to 500 µg/injection (1000 µg/week) of prednisolone phosphate.

The number of mice contained in each of these 3 groups was 2 mice for Control, 2 mice for 250 µg, and 2 mice for 500µg.

The following results were obtained.

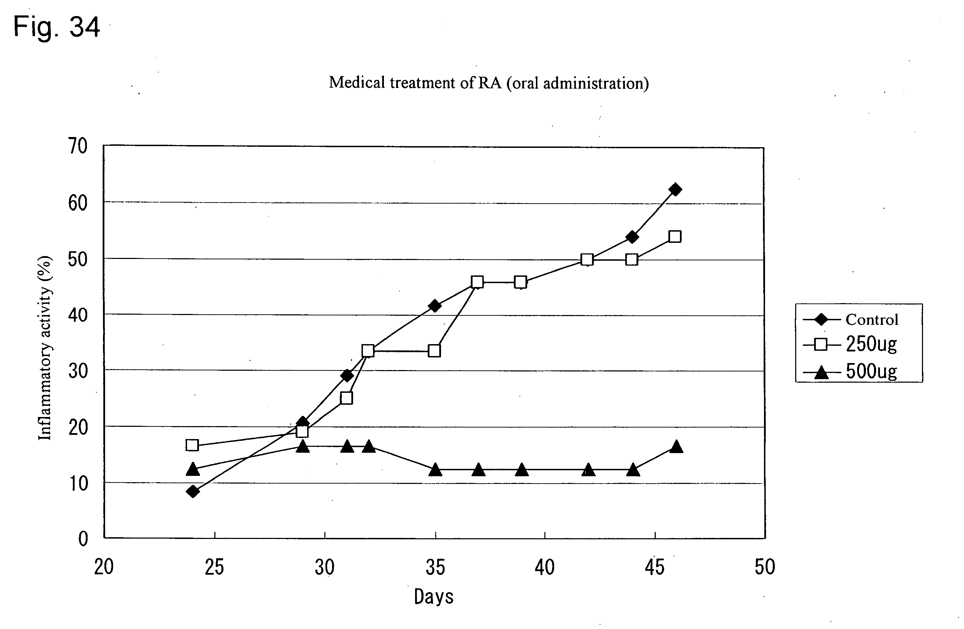

No significant difference was observed between the control and 250 µg groups; and for the 500 µg group, a rise in IA was not observed, and the progression of the inflammation was suppressed (Fig. 34).

The following became clear from this result. It was shown that at least 500 µg/week or more and up to 1000 µg/week of prednisolone phosphate are required for suppressing the progression of inflammation in the mouse model of RA. In the above-described test on medical treatment by intravenous injection, the progression of inflammation in the L-Pred-SLX group was suppressed by using the amount of 10 µg/injection and 20 µg/week prednisolone phosphate. In consideration of this result, the DDS liposome of the present invention seemed to be capable of exhibiting an effect equal to a conventional therapeutic effect, by using up to one-fiftieth of a conventional amount of the drug.

This indicates the possibility that the amount of a drug systemically administered can be reduced, with the drug concentration in lesions maintained, thereby reducing the side effects of the drug, and that when the amount of the drug systemically administered is increased to a concentration that causes no side effect, the drug concentration in lesions can be increased, thereby obtaining higher efficacy of the drug.

### Example 17

### Investigation of Blood Retention of Liposome Treated with 2 Kinds of Hydrophilization

### (1) Preparation of Liposome

Liposome was prepared by using the cholate dialysis based method. More specifically, 46.9 mg of sodium cholate was added to 45.6 mg in total of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain 3 ml of a clear micelle suspension. To this miocelle suspension, PBS buffer solution (pH 7.2) was added to bring up the volume to 5 ml. Then, predonisolone phosphate, completely dissolved in the TAPS buffer solution (pH 8.4) at 2250 mg/6 ml, was added dropwise slowly to this micelle suspension while stirring. After being mixed homogeneously, this micelle suspension containing predonisolone phosphate was subjected to ultrafiltration by using a PM10 membrane (Amicon Co., USA) and the TAPS buffer solution (pH 8.4) to prepare 10 ml of a uniform liposome. The liposome particles in the resulting physiological saline suspension (37°C) was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

### (2) Hydrophilization of Lipid Membrane Surface of Liposomes by Tris(hydroxymethyl)aminomethane

10 ml of the liposome solution prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bonding reaction between the dipalmitoylphosphatidyletanolamine of the lipid on the liposome membrane and the BS3. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris(hydroxymethyl)aminomethane. In this manner, the hydroxyl groups of the tris(hydroxymethyl)aminomethane were coordinated on the dipalmitoylphosphatidyletanolamine of the lipid on the liposome membrane to achieve the hydrophilization of the lipid membrane surface of the liposome.

### (3) Hydrophilization by Cellobiose on Lipid Membrane Surface of Liposome

10 ml of the liposome solution prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bonding reaction between the dipalmitoylphosphatidyletanolamine of the lipid on the liposome membrane and the BS3. This liposome solution was then subjected to ultrafiltration by using an XM300 membrane and a CBS buffer solution (pH 8.5). Then, 50 mg of cellobiose dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and cellobiose. In this manner, the hydroxyl groups of cellobiose were coordinated on the dipalmitoylphosphatidyletanolamine of the lipid on the liposome membrane to achieve the hydrophilization of the lipid membrane surface of the liposome.

### (4) Bonding of Human Serum Albumin (HSA) to Tris(hydroxymethyl)aminomethane Bonded on Liposome Membrane Surface

A coupling reaction method based on a previously reported method (Yamazaki, N., Kodama, M. and Gabius, H. -J. (1994) Methods Enzymol. 242, 56-65) was used. More specifically, the reaction was carried out through a two-stage chemical reaction. That is, 43 mg of sodium metaperiodate dissolved in 1 ml of TAPS buffer solution (pH 8.4) was added to 10 ml of the liposome obtained in (2), and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 µl of 2M NaBH₃CN was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM300 membrane and a CBS buffer solution (pH 8.5).

### (5) Bonding of Human Serum Albumin (HSA) to Cellobiose Bonded on Liposome Membrane Surface

A coupling reaction method based on a previously reported method (Yamazaki, N., Kodama, M. and Gabius, H. -J. (1994) Methods Enzymol. 242, 56-65) was used. More specifically, the reaction was carried out through a two-stage chemical reaction. That is, 43 mg of sodium metaperiodate dissolved in 1 ml of TAPS buffer solution (pH 8.4) was added to 10 ml of the liposome obtained in Example 3, and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 µl of 2M NaBH₃CN was added to the PBS (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM300 membrane and a CBS buffer solution (pH 8.5).

### (6) Hydrophilization of Linker Protein (HSA) by Bonding of Tris(hydroxymethyl)aminomethane on Human Serum Albumin (HSA) bonded to Liposome Membrane Surface

To prepare liposome as one of the hydrophilized samples, 1 mg of 3,3'-dithiobis(sulfosuccinimidyl) propionate (DTSSP; Pierce Co., USA) serving as a crosslinking reagent was added to 1 ml of a part of the liposome solution obtained in (4). The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of hydrophilized liposome in which the DTSSP was bonded to the HSA on the liposome. 13 mg of tris(hydroxymethyl)aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome with the hydrophilized linker protein (HSA), in which tris(hydroxymethyl)aminomethane, human serum albumin and liposome were bonded, (total lipid mass: 2 mg, total protein mass: 200 µg) was obtained as a comparative sample. The liposome particles in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

### (7) Hydrophilization of Linker Protein (HSA) by Bonding of Cellobiose on Human Serum Albumin (HSA) bonded to Liposome Membrane Surface

To prepare liposome as one of the hydrophilized samples, 1 mg of 3,3'-dithiobis(sulfosuccinimidyl) propionate (DTSSP; Pierce Co., USA) serving as a crosslinking reagent was added to 1 ml of a part of the liposome solution obtained in (5). The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of hydrophilized liposome in which the DTSSP was bonded to the HSA on the liposome. 30 mg of cellobiose was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond cellobiose to the DTSSP on the human serum albumin bonded on the liposome membrane surface. In this manner, 2 ml of the liposome with the hydrophilized linker protein (HSA), in which cellobiose, human serum albumin and liposome were bonded, (total lipid mass: 2 mg, total protein mass: 200 µg) was obtained as a hydrophilized sample. The liposome particles in the resulting physiological saline solution (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

### (8) Preparation of Liposome without Hydrophilization

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate and ganglioside (containing 100% of GT1b as glycolipid sugar chain) at a molar ratio of 35:45:5:15, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain 3 ml of a clear micelle suspension. Then, to this micelle suspension, PBS buffer solution (pH 7.2) was added to bring up the volume to 10 ml, and doxorubicin, completely dissolved in the TAPS buffer solution (pH 8.4) at 3 mg/l ml, was added dropwise slowly to this micelle suspension while stirring. After being mixed homogeneously, this micelle suspension containing doxorubicin was subjected to ultrafiltration by using a PM10 membrane (Amicon Co., USA) and the TAPS buffer solution (pH 8.4) to prepare 10 ml of a uniform suspension of the liposome particles without hydrophilization. The liposome particles without hydrophilization in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

### (9) ¹²⁵I-Labeling of Liposome through the Chloramine T Method

A chloramine T (Wako Pure Chemical Co., Japan) solution and a sodium disulfite solution were prepared at 3 mg/ml and 5 mg/ml, respectively. 50 µl of the 3 different types of liposomes prepared in Examples 6 to 8 were put into separate Eppendorf tubes. Then, 15 µl of ¹²⁵I-NaI (NEN Life Science Product, Inc. USA) and 10 µl of chloramine T solution were added thereto and reacted therewith. 10 µl of chloramine T solution was added to the respective solutions every 5 minutes. After 15 minutes from the completion of the above procedure repeated twice, 100 µl of sodium disulfite serving as a reducer was added to the solutions to stop the reaction. Then, each of the resulting solutions was placed on a Sephadex G-50 (Phramacia Biotech. Sweden) column chromatography, and eluted by PBS to purify a labeled compound. Finally, a non-labeled liposome complex was added to each of the solutions to adjust a specific activity (4 × 10⁶ Bq/mg protein). In this manner, three types of ¹²⁵I-labeled liposome solutions were obtained.

### (10) Measurement of the Concentration of Different Types of Liposome in Blood Stream of Mice with Cancer

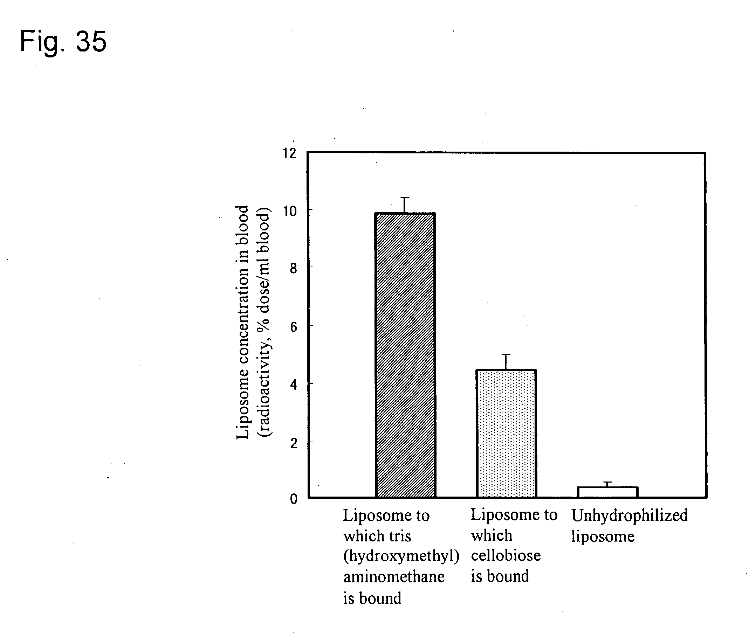

Ehrlich ascites tumor (EAT) cells (about 2 × 10⁷ cells) were implanted subcutaneously into the femoral region in male ddY mice (7 weeks of age), and the mice were used in this test after the tumor tissues grew to 0.3 to 0.6 g (after 6 to 8 days). To this mice with cancer, 0.2 ml of three types of liposome complex labeled with ¹²⁵I according to (9) were injected at a dose of 30 µg/mouse as lipid amount through tail vein. The blood sample was collected after 5 minutes and its radioactivity was measured with a gamma counter (Aloka ARC 300). Distribution of the radioactivity to the blood was expressed as a ratio of the radioactivity per 1 ml of blood to the total radioactivity administered (% administered amount/ml of blood). As shown in Fig. 35, blood retention was increased by the two types of hydrophilization, and among them the liposome hydrophilized with tris(hydroxymethyl)aminomethane had a marked increase in blood retention.

All publications, patents, and patent applications cited herein are incorporated herein by reference in their entirety.

### Industrial Applicability

A targeting liposome for the medical treatment of an inflammatory disease of the present invention is specifically taken into inflammatory lesions and releases a therapeutic drug for the inflammatory disease encapsulated in the liposome. The liposome of the present invention is effective as a therapeutic or diagnostic drug for a drug delivery system for an inflammatory disease.

The liposome modified by a sugar chain such as a sialyl Lewis X sugar chain of the present invention specifically reaches lesions having the expression of E-selectin and P-selectin and releases a drug with therapeutic effects in the lesions. Therefore, the sugar-modified liposome of the present invention is available as a preparation for the medical treatment or diagnosis of an inflammatory disease.

As shown in Examples, when an antiinflammatory drug encapsulated in the targeting liposome for the medical treatment of an inflammatory disease of the present invention is administered, the drug can be accumulated in an inflammatory site at a level a few tens to hundreds of times higher than that of the antiinflammatory drug administered alone and can thus exert a remarkable effect.

## Claims

1. A pharmaceutical composition for the medical treatment or diagnosis of an inflammatory disease comprising a sugar-modified liposome having a sugar chain bound to the membrane of the liposome.

2. The pharmaceutical composition for the medical treatment or diagnosis of an inflammatory disease according to claim 1, wherein lipids constituting the liposome comprise phosphatidylcholines (at a molar ratio of 0 to 70%), phosphatidylethanolamines (at a molar ratio of 0 to 30%), one or more lipids (at a molar ratio of 0 to 30%) selected from the group consisting of phosphatidic acids, long-chain alkyl phosphates, and dicetylphosphoric acids, one or more lipids (at a molar ratio of 0 to 40%) selected from the group consisting of gangliosides, glycolipids, phosphatidylglycerols, and sphingomyelins, and cholesterols (at a molar ratio of 0 to 70%).

3. The pharmaceutical composition for the medical treatment or diagnosis of an inflammatory disease according to claim 2, wherein at least one lipid selected from the group consisting of gangliosides, glycolipids, phosphatidylglycerols, sphingomyelins, and cholesterols assembles to form a raft on the surface of the liposome.

4. The pharmaceutical composition for the medical treatment or diagnosis of an inflammatory disease according to any one of claims 1 to 3, wherein the sugar chain is bound to the membrane of the liposome in a manner that controls the type and density of the sugar chain.

5. The pharmaceutical composition for the medical treatment or diagnosis of an inflammatory disease according to any one of claims 1 to 4, wherein the liposome has a particle size of 30 to 500 nm.

6. The pharmaceutical composition for the medical treatment or diagnosis of an inflammatory disease according to claim 5, wherein the liposome has a particle size of 50 to 300 nm.

7. The pharmaceutical composition for the medical treatment or diagnosis of an inflammatory disease according to any one of claims 1 to 6, wherein the liposome has a zeta potential of -50 to 10 mV.

8. The pharmaceutical composition for the medical treatment or diagnosis of an inflammatory disease according to claim 7, wherein the liposome has a zeta potential of -40 to 0 mV.

9. The pharmaceutical composition for the medical treatment or diagnosis of an inflammatory disease according to claim 8, wherein the liposome has a zeta potential of -30 to -10 mV.

10. The pharmaceutical composition for the medical treatment or diagnosis of an inflammatory disease according to any one of claims 1 to 9, wherein the sugar chain is bound to the membrane of the liposome through a linker protein.

11. The pharmaceutical composition for the medical treatment or diagnosis of an inflammatory disease according to claim 10, wherein the linker protein is an organism-derived protein.

12. The pharmaceutical composition for the medical treatment or diagnosis of an inflammatory disease according to claim 11, wherein the linker protein is a human-derived protein.

13. The pharmaceutical composition for the medical treatment or diagnosis of an inflammatory disease according to claim 12, wherein the linker protein is a human-derived serum protein.

14. The pharmaceutical composition for the medical treatment or diagnosis of an inflammatory disease according to claim 10, wherein the linker protein is human serum albumin or bovine serum albumin.

15. The pharmaceutical composition for the medical treatment or diagnosis of an inflammatory disease according to any one of claims 1 to 14, wherein the linker protein is bound to the raft consisting of at least one lipid selected from the group consisting of gangliosides, glycolipids, phosphatidylglycerols, sphingomyelins, and cholesterols, formed on the surface of the liposome.

16. The pharmaceutical composition for the medical treatment or diagnosis of an inflammatory disease according to any one of claims 1 to 15, wherein the pharmaceutical composition is hydrophilized by bonding a hydrophilic compound to the membrane of the liposome and/or the linker protein.

17. The pharmaceutical composition for the medical treatment or diagnosis of an inflammatory disease according to claim 16, wherein the hydrophilic compound is a substance having a low molecular weight.

18. The pharmaceutical composition for the medical treatment or diagnosis of an inflammatory disease according to claim 16 or 17, wherein the hydrophilic compound is less apt to sterically hinder the sugar chain and does not prevent lectin on the membrane surface of a target cell from proceeding reaction of recognizing the sugar-chain molecule.

19. The pharmaceutical composition for the medical treatment or diagnosis of an inflammatory disease according to any one of claims 16 to 18, wherein the hydrophilic compound has a hydroxyl group.

20. The pharmaceutical composition for the medical treatment or diagnosis of an inflammatory disease according to any one of claims 16 to 19, wherein the hydrophilic compound is any of amino alcohols.

21. The pharmaceutical composition for the medical treatment or diagnosis of an inflammatory disease according to any one of claims 16 to 20, wherein the hydrophilic compound is directly bonded to the membrane surface of the liposome.

22. The pharmaceutical composition for the medical treatment or diagnosis of an inflammatory disease according to claim 16 which is a sugar-modified liposome, wherein the pharmaceutical composition is hydrophilized with a hydrophilic compound represented by the general formula (1):
X-R₁(R₂OH)ₙ (1)
wherein R₁ represents a C₁₋₄₀ linear or branched hydrocarbon chain; R₂ is absent or represents a C₁₋₄₀ linear or branched hydrocarbon chain; X represents a reactive functional group bonded either directly to the lipid of the liposome or the linker protein or to a bivalent crosslinking reagent; and n represents a natural number.

23. The pharmaceutical composition for the medical treatment or diagnosis of an inflammatory disease according to claim 16 which is a sugar-modified liposome, wherein the pharmaceutical composition is hydrophilized with a hydrophilic compound represented by the general formula (2):
H₂N-R₃-(R₄OH)ₙ (2)
wherein R₃ represents a C₁₋₄₀ linear or branched hydrocarbon chain; R₄ is absent or represents a C₁₋₄₀ linear or branched hydrocarbon chain; H₂N represents a reactive functional group bonded either directly to the lipid of the liposome or the linker protein or to a bivalent crosslinking reagent; and n represents a natural number.

24. The pharmaceutical composition for the medical treatment or diagnosis of an inflammatory disease according to claim 16 which is a sugar-modified liposome, wherein the pharmaceutical composition is hydrophilized with a hydrophilic compound represented by the general formula (3):
H₂N-R₅(OH)ₙ (3)
wherein R₅ represents a C₁₋₄₀ linear or branched hydrocarbon chain; H₂N represents a reactive functional group bonded, either directly to the lipid of the liposome or the linker protein or to a bivalent crosslinking reagent; and n represents a natural number.

25. The pharmaceutical composition for the medical treatment or diagnosis of an inflammatory disease according to claim 16, wherein the membrane of the liposome and/or the linker protein are hydrophilized by covalently bonding a hydrophilic compound that is tris(hydroxyalkyl)aminoalkane to the membrane of the liposome and/or the linker protein.

26. The pharmaceutical composition for the medical treatment or diagnosis of an inflammatory disease according to claim 16, wherein the membrane of the liposome and/or the linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, and tris(hydroxypropyl)aminopropane to the membrane of the liposome and/or the linker protein.

27. The pharmaceutical composition for the medical treatment or diagnosis of an inflammatory disease according to any one of claims 1 to 26, wherein the sugar-modified liposome targets lectin selected from the group consisting of C-type lectin comprising selectin, DC-SIGN, DC-SGNR, collectin, and mannose-binding lectin, I-type lectin comprising siglec, P-type lectin comprising a mannose-6-phosphate receptor, R-type lectin, L-type lectin, M-type lectin, and galectin, which serves as a receptor residing on the cell-membrane surface of each tissue.

28. The pharmaceutical composition for the medical treatment or diagnosis of an inflammatory disease according to claim 27, wherein the sugar-modified liposome targets selectin selected from the group consisting of E-selectin, P-selectin, and L-selectin.

29. The pharmaceutical composition for the medical treatment or diagnosis of an inflammatory disease according to any one of claims 1 to 28, wherein the sugar chain is bound to the liposome at a density of 1 to 60 sugar chains per linker protein molecule bound to the liposome.

30. The pharmaceutical composition for the medical treatment or diagnosis of an inflammatory disease according to any one of claims 1 to 28, wherein the sugar chain is bound to the liposome at a density of 1 to 30000 sugar chains per liposome molecule in the case of using the linker protein, and at a maximum density of 1 to 500000 sugar chains per liposome molecule in the case of not using the linker protein.

31. The pharmaceutical composition for the medical treatment or diagnosis of an inflammatory disease according to any one of claims 1 to 30, wherein the sugar chain is selected from the group consisting of Lewis X trisaccharide, sialyl Lewis X tetrasaccharide, 3'-sialyllactosamine trisaccharide, 6'-sialyllactosamine trisaccharide, α-1,2-mannobiose disaccharide, α-1,3-mannobiose disaccharide, α-1,4-mannobiose disaccharide, α=1,6-mannobiose disaccharide, α-1,3/α-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, oligomannose-9 undecasaccharide, lactose disaccharide, 2'-fucosyllactose trisaccharide, difucosyllactose tetrasaccharide, 3-fucosyllactose trisaccharide, 3'-sialyllactose trisaccharide, and 6'-sialyllactose trisaccharide.

32. The pharmaceutical composition for the medical treatment of an inflammatory disease according to any one of claims 1 to 31, wherein the sugar-modified liposome comprises a drug selected from the group consisting of adrenocortical hormones, antiinflammatory drugs, immunosuppressive drugs, anticancer drugs, antimicrobial drugs, antiviral drugs, angiogenesis inhibitors, cytokines, chemokines, anti-cytokine antibodies, anti-chemokine antibodies, anti-cytokine/chemokine receptor antibodies, nucleic acid preparations for therapy using genes such as siRNA and DNA, neuroprotective factors, and antibody drugs.

33. The pharmaceutical composition for the medical treatment of an inflammatory disease according to claim 32, wherein the sugar-modified liposome comprises an adrenocortical hormone or an antiinflammatory drug as the drug.

34. The pharmaceutical composition for the medical treatment of an inflammatory disease according to claim 33, wherein the sugar-modified liposome comprises prednisolone as the drug.

35. The pharmaceutical composition for the medical treatment of an inflammatory disease according to any one of claims 32 to 34, wherein the drug can be accumulated in an inflammatory site at a level 10 or more times higher than that of the drug administered alone.

36. The pharmaceutical composition for the medical treatment or diagnosis of an inflammatory disease according to any one of claims 1 to 35, wherein the inflammatory disease is selected from the group consisting of encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases such as rheumatism, systemic lupus erythematosus, and sarcoidosis, ischemic diseases such as myocardial infarction and cerebral infarction, metabolic diseases such as diabetes and gout, injury, scald, chemical corrosion, and neurodegenerative diseases such as Alzheimer's disease.

37. The pharmaceutical composition for the medical treatment or diagnosis of an inflammatory disease according to claim 36, wherein the inflammatory disease is inflammatory eye disease.

38. The pharmaceutical composition for the medical treatment or diagnosis of an inflammatory disease according to claim 36, wherein the inflammatory disease is rheumatism.

39. The pharmaceutical composition for the medical treatment or diagnosis of an inflammatory disease according to claim 36, wherein the inflammatory disease is enteritis.

40. The pharmaceutical composition for the medical treatment or diagnosis of an inflammatory disease according to any one of claims 1 to 39, wherein the pharmaceutical composition is a pharmaceutical composition for oral administration.

41. The pharmaceutical composition for the medical treatment or diagnosis of an inflammatory disease according to any one of claims 1 to 39, wherein the pharmaceutical composition is a pharmaceutical composition for parenteral administration.

42. A pharmaceutical composition for the medical treatment or diagnosis of an inflammatory disease comprising a liposome having a hydrophilized membrane surface to which no sugar chain is bound.

43. The pharmaceutical composition for the medical treatment or diagnosis of an inflammatory disease according to claim 42, wherein lipids constituting the liposome comprise phosphatidylcholines (at a molar ratio of 0 to 70%), phosphatidylethanolamines (at a molar ratio of 0 to 30%), one or more lipids (at a molar ratio of 0 to 30%) selected from the group consisting of phosphatidic acids, long-chain alkyl phosphates, and dicetylphosphoric acids, one or more lipids (at a molar ratio of 0 to 40%) selected from the group consisting of gangliosides, glycolipids, phosphatidylglycerols, and sphingomyelins, and cholesterols (at a molar ratio of 0 to 70%).

44. The pharmaceutical composition for the medical treatment or diagnosis of an inflammatory disease according to claim 43, wherein the liposome further comprises a protein.

45. The pharmaceutical composition for the medical treatment or diagnosis of an inflammatory disease according to any one of claims 42 to 44, wherein the pharmaceutical composition is hydrophilized by bonding a hydrophilic compound to the membrane of the liposome and/or the linker protein.

46. The pharmaceutical composition for the medical treatment or diagnosis of an inflammatory disease according to claim 45, wherein the hydrophilic compound is a substance having a low molecular weight.

47. The pharmaceutical composition for the medical treatment or diagnosis of an inflammatory disease according to claim 45 or 46, wherein the hydrophilic compound has a hydroxyl group.

48. The pharmaceutical composition for the medical treatment or diagnosis of an inflammatory disease according to any one of claims 45 to 47, wherein the hydrophilic compound is any of amino alcohols.

49. The pharmaceutical composition for the medical treatment or diagnosis of an inflammatory disease according to any one of claims 45 to 48, wherein the hydrophilic compound is directly bonded to the membrane surface of the liposome.

50. The pharmaceutical composition for the medical treatment or diagnosis of an inflammatory disease according to claim 45, wherein the pharmaceutical composition is hydrophilized with a hydrophilic compound represented by the general formula (1):
X-R₁(R₂OH)ₙ (1)
wherein R₁ represents a C₁₋₄₀ linear or branched hydrocarbon chain; R₂ is absent or represents a C₁₋₄₀ linear or branched hydrocarbon chain; X represents a reactive functional group bonded either directly to the lipid of the liposome or the linker protein or to a bivalent crosslinking reagent; and n represents a natural number.

51. The pharmaceutical composition for the medical treatment or diagnosis of an inflammatory disease according to claim 45, wherein the pharmaceutical composition is hydrophilized with a hydrophilic compound represented by the general formula (2):
H₂N-R₃-(R₄OH)ₙ (2)
wherein R₃ represents a C₁₋₄₀ linear or branched hydrocarbon chain; R₄ is absent or represents a C₁₋₄₀ linear or branched hydrocarbon chain; H₂N represents a reactive functional group bonded either directly to the lipid of the liposome or the linker protein or to a bivalent crosslinking reagent; and n represents a natural number.

52. The pharmaceutical composition for the medical treatment or diagnosis of an inflammatory disease according to claim 45, wherein the pharmaceutical composition is hydrophilized with a hydrophilic compound represented by the general formula (3):
H₂N-R₅(OH)ₙ (3)
wherein R₅ represents a C₁₋₄₀ linear or branched hydrocarbon chain; H₂N represents a reactive functional group bonded either directly to the lipid of the liposome or the linker protein or to a bivalent crosslinking reagent; and n represents a natural number.

53. The pharmaceutical composition for the medical treatment or diagnosis of an inflammatory disease according to claim 45, wherein the membrane of the liposome and/or the linker protein are hydrophilized by covalently bonding a hydrophilic compound that is tris(hydroxyalkyl)aminoalkane to the membrane of the liposome and/or the linker protein.

54. The pharmaceutical composition for the medical treatment or diagnosis of an inflammatory disease according to claim 53, wherein the membrane of the liposome and/or the linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, and tris(hydroxypropyl)aminopropane to the membrane of the liposome and/or the linker protein.

55. The pharmaceutical composition for the medical treatment of an inflammatory disease according to any one of claims 42 to 54, wherein the liposome comprises a drug selected from the group consisting of adrenocortical hormones, antiinflammatory drugs, immunosuppressive drugs, anticancer drugs, antimicrobial drugs, antiviral drugs, angiogenesis inhibitors, cytokines, chemokines, anti-cytokine antibodies, anti-chemokine antibodies, anti-cytokine/chemokine receptor antibodies, nucleic acid preparations for therapy using genes such as siRNA and DNA, neuroprotective factors, and antibody drugs.

56. The pharmaceutical composition for the medical treatment of an inflammatory disease according to claim 55, wherein the liposome comprises an adrenocortical hormone or an antiinflammatory drug as the drug.

57. The pharmaceutical composition for the medical treatment of an inflammatory disease according to claim 56, wherein the liposome comprises prednisolone as the drug.

58. The pharmaceutical composition for the medical treatment of an inflammatory disease according to any one of claims 55 to 57, wherein the drug can be accumulated in an inflammatory site at a level 10 or more times higher than that of the drug administered alone.

59. The pharmaceutical composition for the medical treatment or diagnosis of an inflammatory disease according to any one of claims 42 to 58, wherein the inflammatory disease is selected from the group consisting of encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases such as rheumatism, systemic lupus erythematosus, and sarcoidosis, ischemic disease such as myocardial infarction and cerebral infarction, metabolic diseases such as diabetes and gout, injury, scald, chemical corrosion, and neurodegenerative disease such as Alzheimer's disease.

60. The pharmaceutical composition for the medical treatment or diagnosis of an inflammatory disease according to claim 59, wherein the inflammatory disease is inflammatory eye disease.

61. The pharmaceutical composition for the medical treatment or diagnosis of an inflammatory disease according to claim 59, wherein the inflammatory disease is rheumatism.

62. The pharmaceutical composition for the medical treatment or diagnosis of an inflammatory disease according to claim 59, wherein the inflammatory disease is enteritis.

63. The pharmaceutical composition for the medical treatment or diagnosis of an inflammatory disease according to any one of claims 42 to 62, wherein the pharmaceutical composition is a pharmaceutical composition for oral administration.

64. The pharmaceutical composition for the medical treatment or diagnosis of an inflammatory disease according to any one of claims 42 to 62, wherein the pharmaceutical composition is a pharmaceutical composition for parenteral administration.
